# EUROPEAN PATENT SPECIFICATION

(11) **EP 2 567 241 B1**
(45) Date of publication and mention of the grant of the patent: **04.11.2015**
(21) Application number: 11778172.4
(22) Date of filing: 03.05.2011
(51) Int. Cl.: G01N 33/92, G01N 33/15, G06F 17/10

(54) **DETECTION AND MONITORING OF NONALCOHOLIC FATTY LIVER DISEASE**
ERKENNUNG UND ÜBERWACHUNG VON NICHT ALKOHOLBEDINGTER FETTLEBER
DÉTECTION ET SUIVI D'UNE HÉPATITE GRAISSEUSE NON ALCOOLIQUE

(30) Priority: 03.05.2010 US 330579 P; 24.06.2010 US 358190 P
(43) Date of publication of application: 13.03.2013
(73) Proprietor: THE CLEVELAND CLINIC FOUNDATION, Cleveland, OH 44195 (US)
(72) Inventor: FELDSTEIN, Ariel, E., Highland Heights OH 44143 (US); HANZEN, Stanley, L., Pepper Pike OH 44124 (US)
(74) Representative: Maiwald Patentanwalts GmbH
(86) International application number: PCT/US2011/035008
(87) International publication number: WO 2011/140093

(56) References cited:
- WO-A1-2007/136674
- US-A1- 2007 225 919
- US-A1- 2008 311 593
- NOMOTO KAZUHIRO ET AL: "Cytoplasmic fine granular expression of 8-hydroxydeoxyguanosine reflects early mitochondrial oxidative DNA damage in nonalcoholic fatty liver disease", APPLIED IMMUNOHISTOCHEMSITRY AND MOLECULAR MORPHOLOGY, vol. 16, no. 1, 1 January 2008 (2008-01-01), pages 71-75, XP009161345, LIPPINCOTT WILLIAMS & WILKINS, US ISSN: 1541-2016
- HASHIMOTO ETSUKO, FARRELL GEOFFREY: "Will non-invasive markers replace liver biopsy for diagnosing and staging fibrosis in non-alcoholic steatohepatitis?", JOURNAL OF GASTROENTEROLOGY AND HEPATOLOGY, vol. 24, 30 March 2009 (2009-03-30), pages 501-503, XP002713265, DOI: 10.1111/j.1440-1746.2009.05806.x
- MANGESH PAGADALA ET AL: "Predictors of Steatohepatitis and Advanced Fibrosis in Non-Alcoholic Fatty Liver Disease", CLINICS IN LIVER DISEASE, vol. 13, no. 4, 1 November 2009 (2009-11-01), pages 591-606, XP055080121, ISSN: 1089-3261, DOI: 10.1016/j.cld.2009.07.011
- ANGULO PAUL ET AL: "Independent predictors of liver fibrosis in patients with nonalcoholic steatohepatitis", HEPATOLOGY, vol. 30, no. 6, December 1999 (1999-12), pages 1356-1362, XP002713266, ISSN: 0270-9139
- BHATTACHARYYA T ET AL: "59 Mass Spectrometric Detection of Lipid Oxidation Products in Murine and Human Nonalcoholic Fatty Liver Disease", GASTROENTEROLOGY, vol. 134, no. 4, 1 April 2008 (2008-04-01) , page A-753, XP023435314, ELSEVIER, PHILADELPHIA, PA ISSN: 0016-5085, DOI: 10.1016/S0016-5085(08)63517-9 [retrieved on 2008-04-01]
- CAMPOS GUILHERME M; BAMBHA KIRAN; VITTINGHOFF ERIC; RABL CHARLOTTE;: "A clinical scoring system for predicting nonalcoholic steatohepatitis in morbidly obese patients", HEPATOLOGY, vol. 47, no. 6, 1 June 2008 (2008-06-01), page 1916, XP002713267, ISSN: 0270-9139
- POYNARD THIERRY ET AL: "Diagnostic value of biochemical markers (NashTest) for the prediction of non alcoholo steato hepatitis in patients with non-alcoholic fatty liver disease", BMC GASTROENTEROLOGY, vol. 6, no. 1, 10 November 2006 (2006-11-10), page 34, XP021022807, BIOMED CENTRAL LTD., LONDON, GB ISSN: 1471-230X, DOI: 10.1186/1471-230X-6-34
- NAGA CHALASANI ET AL: "Systemic Levels of Lipid Peroxidation and Its Metabolic and Dietary Correlates in Patients with Nonalcoholic Steatohepatitis", AMERICAN JOURNAL OF GASTROENTEROLOGY, vol. 99, no. 8, 1 August 2004 (2004-08-01) , pages 1497-1502, XP055080115, ISSN: 0002-9270, DOI: 10.1111/j.1572-0241.2004.30159.x
- FELDSTEIN, A, E. ET AL.: 'MASS SPECTROMETRIC PROFILING OF OXIDIZED LIPID PRO DUCTS IN HUMAN NONALCOHOLIC FATTY LIVER DISEASE AND NONALCOHOLIC STEATOHEPAT ITIS' JOURNAL OF LIPID REASEARCH vol. 51, no. 10, 14 July 2010, pages 3046 - 3054

## Description

### TECHNICAL FIELD

This application relates to a method of assessing the severity of Nonalcoholic Fatty Liver Disease (NAFLD) in a subject, and more particularly relates to a method of detecting, diagnosing, and/or monitoring Nonalcoholic steatohepatitis (NASH) and/or liver fibrosis in a subject with or suspected of having NAFLD.

### BACKGROUND

Nonalcoholic Fatty Liver Disease (NAFLD) is currently the most common form of chronic liver disease affecting both adults and children, and is strongly associated with obesity and insulin resistance. One in three adults and one in ten children or adolescents in the United States have hepatic steatosis, a stage within the spectrum of NAFLD, that is characterized by triglyceride accumulation in liver cells and follows a benign non-progressive clinical course. Nonalcoholic steatohepatitis (NASH) is defined as lipid accumulation with evidence of cellular damage, inflammation, and different degrees of scarring or fibrosis. NASH is a serious condition as approximately 25% of these patients progress to cirrhosis and its feared complications of portal hypertension, liver failure and hepatocellular carcinoma.

At present, the available non-invasive markers for NAFLD include a set of clinical signs and symptoms, non-specific laboratory, and radiological imaging tests and combinations of clinical and blood test results. Although, several of these markers are in general useful for the diagnostic evaluation of a patient with suspected NAFLD, they lack specificity and sensitivity to distinguish NAFLD from NASH and determine the presence and stage of fibrosis. This represents a key clinical problem because patients with NASH and fibrosis are probably those who need close monitoring and follow up, and are the potential targets for therapeutic intervention when specific treatments for this condition become available. To date, liver biopsy, an invasive procedure, remains the gold standard for NAFLD diagnosis.

Bhattacharyya et al., 59 mass spectrometric detection of lipid oxidation products in murine and human nonalcoholic fatty liver disease, Gastroenterology, Elsevier, Philadelphia, PA, vol 134, no 4, 1 April 2008 pages A-753, have described a set of non-invasive markers for nonalcoholic fatty liver disease by measuring several fatty acid oxidation products in plasma and liver tissue.

Therefore, there is a great need for development of noninvasive methods that can reliably identify patients with NASH and stage the magnitude of fibrosis present.

### SUMMARY

The present invention is defined by a method of predicting, detecting, or monitoring nonalcoholic steatohepatitis in a subject with or suspected of having nonalcoholic fatty liver disease, the method comprising: analysing a bodily sample obtained from blood, serum or plasma from the subject, the sample including at least one oxidized fatty acid product; determining level of the at least one oxidized fatty acid product in the sample; and deriving a risk score using the determined level, wherein an increased risk score compared to a control is indicative of an increased severity or risk of nonalcoholic steatohepatitis, wherein the risk score is derived using an analytical process, the analytical process using a dataset that includes the determined level of the at least one oxidized fatty acid product and quantitative data from one or more clinical indicia including at least one of the subject's age, body mass index, or concentration of aspartate transaminase or alanine transaminase.

In a further preferred embodiment, the one or more clinical indicia comprise at least two of the subject's age, body mass index, and concentration of aspartate transaminase or alanine transaminase.

In a further preferred embodiment, the dataset includes the determined level of the at least one oxidized fatty acid product, the subject's age, body mass index, and concentration of aspartate transaminase or alanine transaminase.

In a further preferred embodiment, the analytical process comprises using a Linear Discriminant Analysis model, a support vector machine classification algorithm, a recursive feature elimination model, a prediction analysis of microarray model, a Logistic Regression model, a CART algorithm, a FlexTree algorithm, a random forest algorithm, a MART algorithm, or Machine Learning algorithms.

In a further preferred embodiment, the process comprises using a Linear Discriminant Analysis model or a Logistic Regression model, and said model comprises terms for the dataset selected to provide a quality metric greater than about 0.8 for an increased severity or risk of nonalcoholic steatohepatitis.

In a further preferred embodiment, the method further comprises obtaining a plurality of risk scores for a plurality of samples obtained at a plurality of different times from the subject.

In a further preferred embodiment, the statistical significance (p value) of the level of at least one oxidized fatty acid product in a subject with nonalcoholic steatohepatitis compared to a level in a normal subject is less than 0.2.

In a further preferred embodiment, the p value is less than about 0.05.

In a further preferred embodiment, the at least one oxidized fatty acid product is selected from the group consisting of 12-HETE, 15-HETE, 11-HETE, 8-HETE, 9-HETE, 5-HETE, 13-HODE, 9-HODE, 9-oxoODE, 13-oxoODE, and F2isoprostanes.

In a further preferred embodiment, the at least one oxidized fatty acid product is selected from the group consisting of 13-HODE, 9-HODE, 9-oxoODE, and 13-oxoODE.

In a further preferred embodiment, the method further comprises comparing the ratio of at least one oxidized fatty acid product to at least one oxidized fatty acid product precursor molecule.

In a further preferred embodiment, the at least one oxidized fatty acid product precursor molecule comprises arachidonic acid or linoleic acid.

In a further preferred embodiment, the control comprises the risk score of a normal or healthy subject or tissue.

In a further preferred embodiment, the analytical process for determining the risk score comprises the algorithm: risk score = [-10.051 + 0.0463*Age(years) + 0.147*BMI(kg/m²) + 0.0293*(AST or ALT)(IU/L) + 2.658*(Oxidized fatty acid product: Oxidized fatty acid precursor Ratio(mmol/mol)] * 10.

In a further preferred embodiment, the analytical process for determining the risk score comprises the algorithm: risk score = [-10.051 + 0.0463*Age(years) + 0.147*BMI(kg/m²) + 0.0293*AST(IU/L) + 2.658*HODE-13:LA Ratio(mmol/mol)] * 10.

In a further preferred embodiment, a risk score of at 2.2 indicates that the subject has or a substantially increased risk of NASH.

### BRIEF DESCRIPTION OF THE DRAWINGS

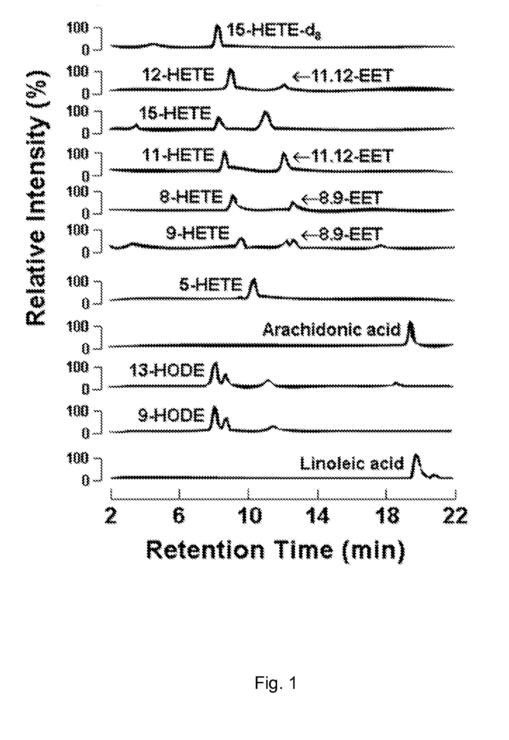

Fig. 1 illustrates a plot showing detection and quantification of oxFA profile by ESI/LC/MS/MS. Individual isomers of HETEs, EETs, HODEs and oxoODEs formed by oxidation of arachidonic acid and linoleic acid are quantified with one single injection. Lipid extracts are resolved by HPLC and monitored on-line by electrospray ionization tandem mass spectrometry as detailed under the Methods section. Abbreviations: EETs: epoxy eicosatetraenoic acid; HETE: hydroxy eicosatetraenoic acid; HODE: hydroxy octadecadenoic acid; 15-HETE-d8, internal standard.

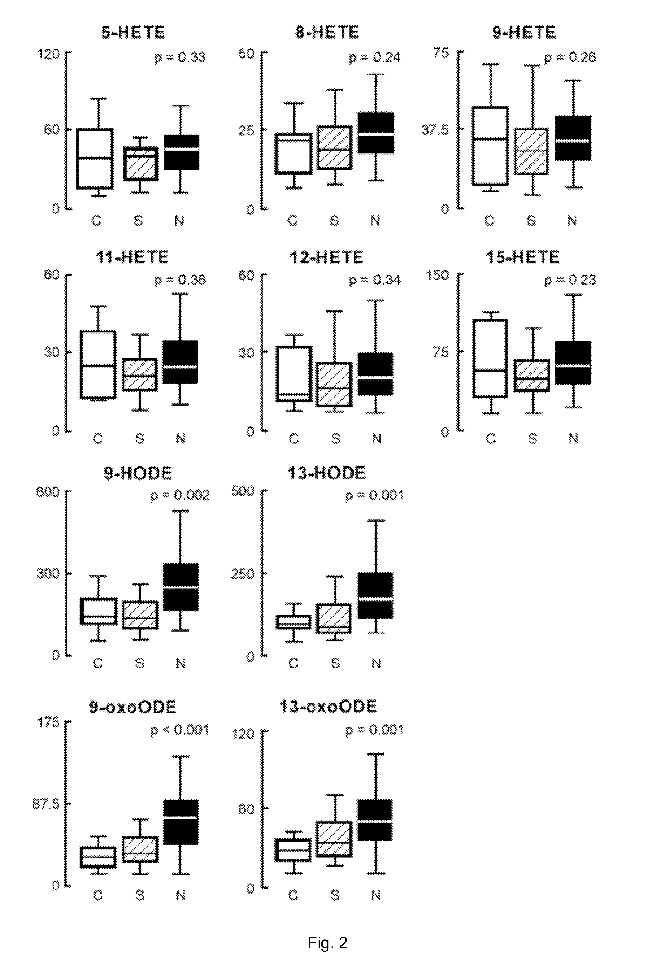

Fig. 2 illustrates box-whisker plots showing OxFA levels are markedly increased in the blood of patients with NASH. The box-whisker plots are represented with the lower boundary of the box indicating the 25th percentile, the line within the box indicating the median value, and the upper boundary of the box indicating the 75th percentile. The whiskers extend to the most extreme data point which is no more than 1.5 times the interquartile range from the box. P values represent differences among groups. Abbreviations: HETE: hydroxy-eicosatetraenoic acid; HODE: hydroxy-octadecadienoic acid; oxoODE: oxo-octadecadienoic acid; pmol/mL: picomoles per milliliter.

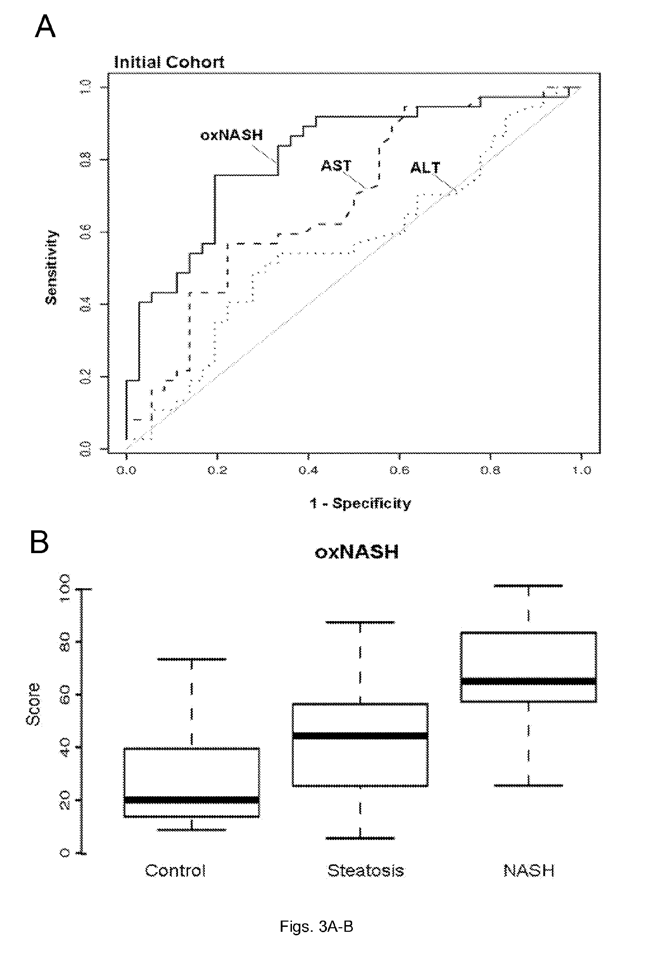

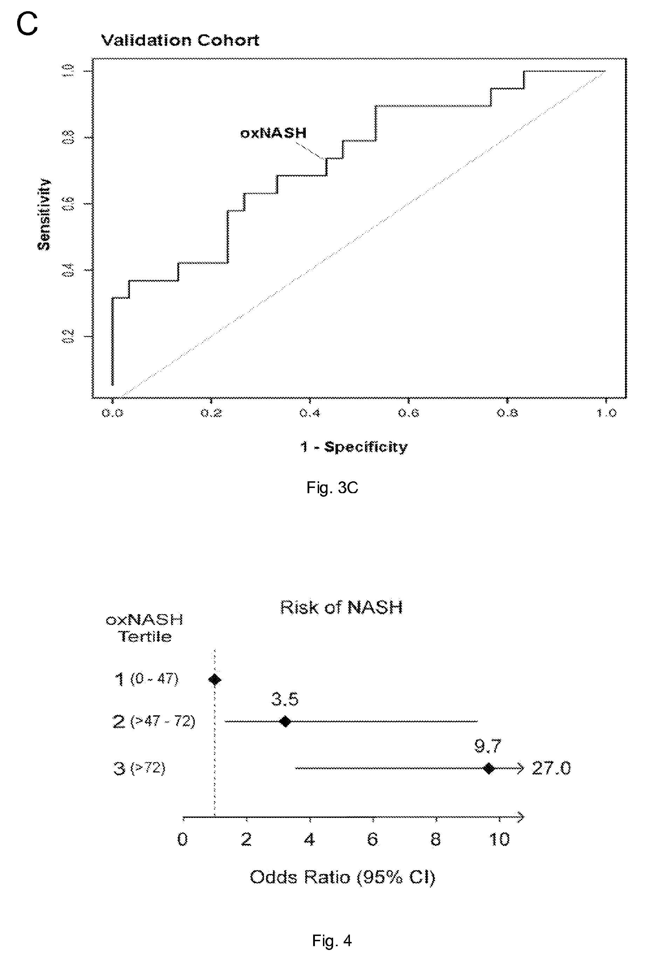

Fig. 3 illustrates plots showing circulating oxFA levels as predictors of NASH in patients with suspected NAFLD. A scoring system (oxNASH) that included 13-HODE/LA ratio, Age, BMI and AST showed best prediction of NASH diagnosis. (A) The AUC curve on the initial training cohort (n = 73) for oxNASH was estimated to be 0.83 (95% CI: 0.73, 0.93) and was found to be significantly higher than the AUCs of both that of serum AST 0.69 (95% CI: 0.56, 0.81) and serum ALT 0.56 (95% CI: 0.43, 0.70) (p<0.01). (B) The box-whisker plot for oxNASH in the three groups of patients is represented with the lower boundary of the box indicating the 25th percentile, the line within the box indicating the median value, and the upper boundary of the box indicating the 75th percentile. The whiskers extend to the most extreme data point which is no more than 1.5 times the interquartile range from the box. (C) The AUC in the independent validation group (n = 49) was estimated to be 0.74 (95% CI: 0.6, 0.88). Abbreviations: AUC: area under the curve; 95% CI: ninety five percent confidence interval; AST: aspartate transaminase; ALT: alanine transaminase.
Fig. 4 illustrates a forest plot illustrating the odds ratio and 95 confidence intervals for risk of histopathologic diagnosis of NASH based upon oxNASH tertiles. Numbers in parentheses represent oxNASH tertile ranges. For each comparison, the first tertile served as the reference group.

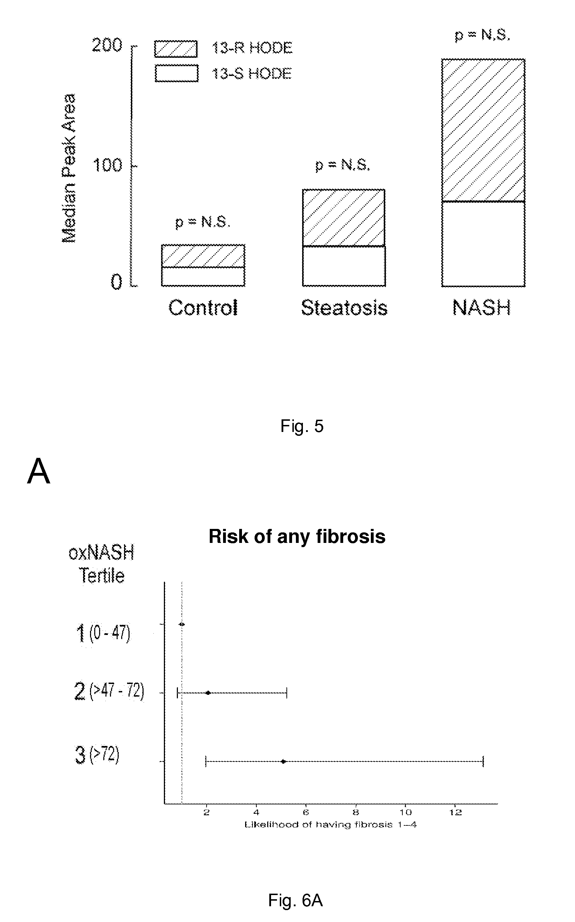

Fig. 5 illustrates a chart showing free radical mediated processes are key mediators of lipid oxidation in NAFLD. Specific oxidized lipid species were separated by liquid chromatography on a chiral phase to identify and quantify the structural isomers and their chiral distribution. A significant increase in peak area of both 13(S) HODE and 13(R) HODE were observed in patients with NASH as compared to patients with hepatic steatosis and patients with normal liver biopsy. The peak area of 13(S) HODE was similar to that of 13 (R) HODE in the three groups of patients.

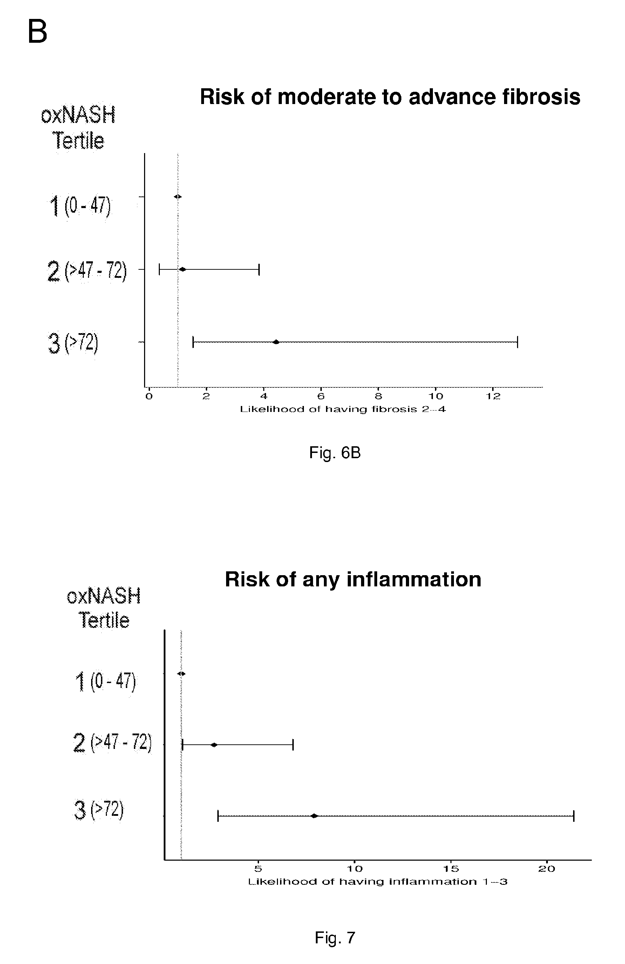

Fig. 6 illustrates a forest plot showing the odds ratio and 95 confidence intervals for risk of having any fibrosis (stage 1 to 4) or moderate to advance fibrosis (stage 2 to 4) based upon oxNASH tertiles. The risk of having any fibrosis on liver biopsy for subjects with oxNASH levels in the second or third tertile versus the lowest tertile was 2.1-fold and 5.1-fold higher odds, respectively. The risk of having moderate to advance fibrosis on liver biopsy for subjects with oxNASH levels in the second or third tertile versus the lowest tertile was 1.2-fold and 4.4-fold higher odds, respectively.
Fig. 7 illustrates a forest plot showing the odds ratio and 95 confidence intervals for risk of having histopathological inflammation on liver biopsy (grade 1 to 3) based upon oxNASH tertiles. The risk of having inflammation on liver biopsy for subjects with oxNASH levels in the second or third tertile versus the lowest tertile was 2.7-fold and 7.9-fold higher odds, respectively.

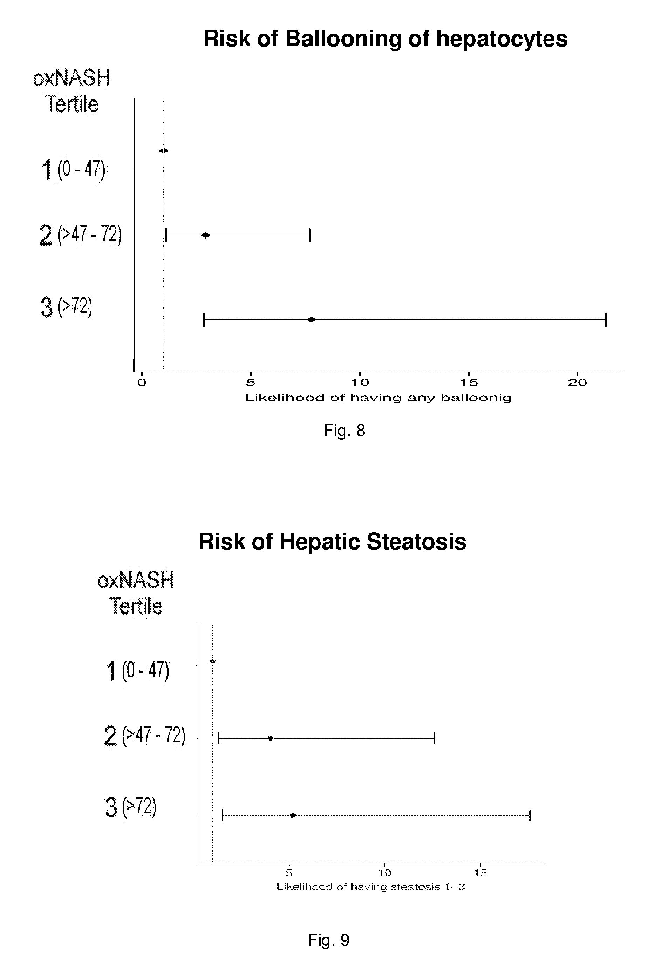

Fig. 8 illustrates a forest plot showing the odds ratio and 95 confidence intervals for risk of having any hepatocyte ballooning degeneration on liver biopsy based upon oxNASH tertiles. The risk of having ballooning of hepatocytes on liver biopsy for subjects with oxNASH levels in the second or third tertile versus the lowest tertile was 2.9-fold and 7.8-fold higher odds, respectively. For each comparison, the first tertile served as the reference group.
Fig. 9 illustrates a forest plot showing the odds ratio and 95 confidence intervals for risk of having hepatic steatosis (grade 1 to 3) on liver biopsy based upon oxNASH tertiles. The risk of having steatosis on liver biopsy for subjects with oxNASH levels in the second or third tertile versus the lowest tertile was 4.0-fold and 5.2-fold higher odds, respectively.

### DETAILED DESCRIPTION

Unless specifically addressed herein, all terms used have the same meaning as would be understood by those of skilled in the art of the present invention. The following definitions will provide clarity with respect to the terms used in the specification and claims to describe the present invention.

The term "monitoring" as used herein refers to the use of results generated from datasets to provide useful information about an individual or an individual's health or disease status. "Monitoring" can include, for example, determination of prognosis, risk-stratification, selection of drug therapy, assessment of ongoing drug therapy, determination of effectiveness of treatment, prediction of outcomes, determination of response to therapy, diagnosis of a disease or disease complication, following of progression of a disease or providing any information relating to a patient's health status over time, selecting patients most likely to benefit from experimental therapies with known molecular mechanisms of action, selecting patients most likely to benefit from approved drugs with known molecular mechanisms where that mechanism may be important in a small subset of a disease for which the medication may not have a label, screening a patient population to help decide on a more invasive/expensive test, for example, a cascade of tests from a non-invasive blood test to a more invasive option such as biopsy, or testing to assess side effects of drugs used to treat another indication.

The term "quantitative data" as used herein refers to data associated with any dataset components (*e.g.*, markers, clinical indicia, metabolic measures, or genetic assays) that can be assigned a numerical value. Quantitative data can be a measure of the level of a marker and expressed in units of measurement, such as molar concentration, concentration by weight, etc. For example, if the marker is an oxidized fatty acid product, quantitative data for that marker can be oxidized fatty acid products measured using methods known to those skilled in the art and expressed in mM or mg/dL concentration units.

The term "subject" as used herein relates to an animal, such as a mammal including a small mammal *(e.g.,* mouse, rat, rabbit, or cat) or a larger mammal *(e.g.,* dog, pig, or human). In particular aspects, the subject is a large mammal, such as a human, that is suspected of having or at risk of NASH.

The term "diagnosing NASH" as used herein refers to a process aimed at one or more of: determining if a subject is afflicted with NASH; determining the severity or stage of NASH related pathologies in a subject; determining the risk that a subject is afflicted with NASH; and determining the prognosis of a subject afflicted with NASH.

The term "bodily sample" is used herein in its broadest sense. A bodily sample may be obtained from a subject *(e.g.,* a human) or from components *(e.g.,* tissues) of a subject. The sample may be of any biological tissue or fluid with which biomarkers described herein may be assayed. Frequently, the sample will be a "clinical sample", *i.e.,* a sample derived from a patient. Such samples includeblood, blood plasma or blood serum. The term biological sample also encompasses any material derived by processing the biological sample Processing of the bodily sample may involve one or more of, filtration, distillation, extraction, concentration, inactivation of interfering components, addition of reagents, and the like.

The terms "normal" and "healthy" are used herein interchangeably. They refer to an individual or group of individuals who have not shown any symptoms of NASH, such as liver inflammation, fibrosis, steatosis, and have not been diagnosed with NASH. Preferably, the normal individual (or group of individuals) is not on medication affecting NASH. In certain embodiments, normal individuals have similar sex, age, body mass index as compared with the individual from which the sample to be tested was obtained. The term "normal" is also used herein to qualify a sample isolated from a healthy individual.

The terms "control" or "control sample" as used herein refer to one or more biological samples isolated from an individual or group of individuals that are normal (*i.e.*, healthy). The term "control", "control value" or "control sample" can also refer to the compilation of data derived from samples of one or more individuals classified as normal, one or more individuals diagnosed with nonalcoholic fatty liver disease, one or more individuals diagnosed with NASH, one or more individuals diagnosed with hepatic steatosis, and/or one or more individuals diagnosed with liver fibrosis.

The term "indicative of NASH" as used herein, when applied to an amount of at least one OxFA in a sample, refers to a level or an amount, which is diagnostic of NASH such that the level is found significantly more often in subjects with the disease than in patients without the disease or another stage of nonalcoholic fatty liver disease, such as hepatic steatosis (as determined using routine statistical methods setting confidence levels at a minimum of 95%). Preferably, a level, which is indicative of NASH, is found in at least about 60% of patients who have the disease and is found in less than about 10% of subjects who do not have the disease. More preferably, a level, which is indicative of NASH, is found in at least about 70%, at least about 75%, at least about 80%, at least about 85%, at least about 90%, at least about 95% or more in patients who have the disease and is found in less than about 10%, less than about 8%, less than about 5%, less than about 2.5%, or less than about 1% of subjects who do not have the disease.

The terms "mass spectrometry" or "MS" as used herein refer to methods of filtering, detecting, and measuring ions based on their mass-to-charge ratio, or "m/z." In general, one or more molecules of interest are ionized, and the ions are subsequently introduced into a mass spectrographic instrument where, due to a combination of magnetic and electric fields, the ions follow a path in space that is dependent upon mass ("m") and charge ("z").

The term "ionization" as used herein refers to the process of generating an analyte ion having a net electrical charge equal to one or more electron units. Negative ions are those having a net negative charge of one or more electron units, while positive ions are those having a net positive charge of one or more electron units.

This application relates to the identification and use of fatty acid oxidation products as systemic non-invasive (*e.g*., plasma) markers to diagnose, identify, stage, and monitor nonalcoholic fatty liver disease (NAFLD), nonalcoholic steatohepatitis (NASH), fibrosis of the liver, steatosis of the liver, inflammation of the liver, and ballooning of the liver in subjects suspected of having NAFLD. Using a highly sensitive and specific tandem mass spectrometry approach, it was found that specific oxidized fatty acid products (OxFAs) are markedly increased in the blood of patients with NASH and are mainly the result of free radical mediated processes. The findings also show that the amounts or levels of these oxidation products correlate with pathologies, such as the presence of diabetes or hypertension, associated with NASH and severity of liver disease independent of other metabolic factors suggesting liver-specificity for the source of these products in blood. It was also found that a scoring system derived from the amount of at least one OxFA *(e.g.,* 13-hydroxy-octadecadienoic acid) and other clinical indicia *(e.g.,* age, BMI and aspartate transaminase (AST) or alanine transaminase of a subject) can be useful in predicting, detecting, monitoring, or classifying NASH, fibrosis of the liver, steatosis of the liver, inflammation of the liver, and ballooning of the liver. Moreover, the scoring system can be used in a method of detecting or monitoring the efficacy of a therapeutic agent administered to a subject for treating fibrosis of the liver or NASH.

OxFAs present in a bodily sample obtained from a subject can be derived from both enzymatic and non-enzymatic free radical mediated processes and include, but are not limited to, hydroxyl-octadecadienoic acids (HODEs), hydroxyl-eicosatetraenoic acids (HETEs), oxo-octadecadienoic acids (oxoODEs), and isoprostanes.

In some aspects, the at least one OxFA in a bodily sample is a product of free radical-mediated oxidation of linoleic acid or conguated linoleic acid including, but not limited to, 9-HODE, 13-HODE, 9-oxoODE and 13-oxoODE. The at least one OxFA present in a sample obtained from a subject can also include products of free radical-mediated oxidation of arachidonic acid including, but not limited to, 5-HETE, 8-HETE, 9-HETE, 11-HETE, 12-HETE and 15-HETE. In other aspects, the bodily sample further includes at least one OxFA and at least one corresponding precursor molecule (*e.g.*, linoleic acid or arachidonic acid).

In some aspects, the at least one OxFA in a sample includes all structural isomers of an individual OxFA. For example, the OxFA 13-HODE in a sample may include both 13(S)-HODE and 13(R)-HODE isoforms of 13-HODE.

In other aspects, the statistical significance (p value) of the level of at least one oxidized fatty acid product in a subject with nonalcoholic steatohepatitis compared to a level in a normal subject is less than 0.2. In other aspects, the p value can be less than about 0.05. Examples of oxidized fatty acid products whose levels in a subject with nonalcoholic steatohepatitis compared to a level in a normal subject have a statistical significance less than *0.2* (*i.e.,* p value less 0.2) are 9-HODE, 13-HODE, 9-oxoODE and 13-oxoODE.

In still other aspects, the oxidized fatty acid product can include isoprostanes, such as F2isoprostane. Isoprostanes are a complex family of compounds produced from arachidonic acid via a free-radical-catalyzed mechanism. They can be quantified as reliable markers of lipid peroxidation.

The bodily sample obtained from the subject can potentially include body fluids, such as blood, plasma, or serum.

In accordance with an aspect of the application, the bodily sample can comprise a blood sample obtained non-invasively from the subject. In some aspects, the amount of blood taken from a subject is about 0.1 ml or more. In an exemplary embodiment, the bodily sample is blood plasma isolated from a whole blood sample obtained from a subject. Blood plasma may be isolated from whole blood using well known methods, such as centrifugation.

The bodily samples can be obtained from the subject using sampling devices, such as syringes, swabs or other sampling devices used to obtain liquid and/or solid bodily samples non-invasively. These samples can then be stored in storage containers. The storage containers used to contain the collected sample can comprise a non-surface reactive material, such as polypropylene. The storage containers should generally not be made from untreated glass or other sample reactive material to prevent the sample from becoming absorbed or adsorbed by surfaces of the glass container.

Collected samples stored in the container may be stored under refrigeration temperature. For longer storage times, the collected sample can be frozen to retard decomposition and facilitate storage. For example, samples obtained from the subject can be stored in a falcon tube and cooled to a temperature of about -80°C. The collected bodily sample can be stored in the presence of a chelating agent, such as ethylenediaminetetraacetic acid (EDTA). The collected bodily sample can also be stored in the presence of an antioxidant, such as butylated hydroxytoluene (BHT) or diethylenetriamine pentaacetic acid, and/or kept in an inert atmosphere (*e.g.*, overlaid with argon) to inhibit oxidation of the sample.

Bodily samples obtained from the subject can then be contacted with a solvent, such as an organic solvent, to isolate and/or separate lipids including OxFAs from the bodily sample. In one aspect, the lipids can be extracted or separated from the bodily sample by contacting the bodily sample with at least one organic solvent under conditions such that an extracted sample is produced. The solvent can include any chemical useful for the removal *(i.e.,* extraction) of a lipid from a bodily sample. For example, where the bodily sample comprises plasma, the solvent can include a water/methanol mixture. It will be appreciated by one skilled in the art that the solvent is not strictly limited to this context, as the solvent may be used for the removal of lipids from a liquid mixture, with which the liquid is immiscible in the solvent. Those skilled in the art will further understand and appreciate other appropriate solvents that can be employed to extract lipids from the bodily sample.

The solvent can include solvent mixtures comprising miscible, partially miscible, and/or immiscible solvents. For example, the solvent can comprise a mixture of water and methanol. The solvent can also be combined with other solvents or liquids, which are not useful for the removal of the lipids. The other solvents in the solvent mixture can act as carriers, which facilitate mixing of the solvent with the bodily sample or transfer of the extracted lipids from the bodily sample.

In another aspect, the bodily sample can be extracted in the presence of a chelating agent, such as ethylenediaminetetraacetic acid (EDTA), and/or an antioxidant, such as butylated hydroxytoluene (BHT), to inhibit oxidation of the sample and extracted lipid.

Following solvent extraction, the at least one OxFA in a sample can be further purified by liquid chromatography (LC) prior to quantification using mass spectrometry. Liquid chromatography removes impurities and may be used to concentrate the OxFAs for detection. Traditional LC relies on chemical interactions between sample components (*e.g.*, OxFAs) and a stationary phase such as a column packing. Laminar flow of the sample, mixed with a mobile phase, through the column is the basis for separation of the components of interest. The skilled artisan understands that separation in such columns is a partition process.

In various embodiments, one or more of the purification and/or analysis steps can be performed in an automated fashion. By careful selection of valves and connector plumbing, two or more chromatography columns can be connected as needed such that material is passed from one to the next without the need for any manual steps. In certain embodiments, the selection of valves and plumbing is controlled by a computer pre-programmed to perform the necessary steps. The chromatography system can also be connected in-line to the detector system, *e.g.*, an MS system. Thus, an operator may place a tray of purified samples in an autosampler, and the remaining operations are performed under computer control, resulting in purification and analysis of all samples selected. In one embodiment, a diverter valve is placed in-line between the LC column and the interface with the MS. The diverter valve directs the LC effluent into a waste container until slightly prior to the time expected peak retention). This prevents the solvent front and other impurities from being passed into the MS device.

As used here, "in-line" refers to a configuration in which the LC and the ionization/injection device for the first MS quadropole are functionally connected in order that the LC effluent passes directly into the first MS device. "In-line" configurations may include a selector valve such that the effluent from two or more LC columns may be directed individually into the MS device and, optionally, to a waste container. Such a configuration is useful for a high throughput system and reduces the analysis time required for a large number of samples. High throughput systems may be designed in which an autosampler initiates LC purifications on the two or more LC columns at staggered intervals. In this way, the purified OxFA peak is eluted from each LC column at a known interval. In certain embodiments, the purified OxFA peaks eluting from the two or more LC columns are directed into the MS device in rapid succession, but with sufficient temporal separation that individual measurements are not compromised. Such a high throughput system reduces the amount of "idle-time" for MS detection attributable to the LC procedure, which typically requires more time than the MS analysis.

By contrast, "off-line" refers to a configuration that requires manual intervention to transfer the LC effluent to the MS device. Typically, the LC effluent is captured by a fractionator and must be manually loaded into a MS device or into an autosampler for subsequent MS detection. Off-line configurations are useful, but less desirable because of the increased time required to process large numbers of samples.

The at least one OxFA purified by LC is conveniently detected and quantified by mass spectrometry (MS). The OxFA containing effluent from the LC is injected into an ionization chamber of the MS in which a first (parent) ion is produced. The parent ion may be detected directly in a first MS, or it may be isolated by the first MS, fragmented into characteristic daughter ions, and one or more of the daughter ions detected in a second MS *(i.e.,* tandem MS).

The ions may be detected using several detection modes. For example, selected ions may be detected using a selective ion monitoring mode (SIM) which includes multiple reaction monitoring (MRM) or selected reaction monitoring (SRM). Alternatively, ions may be detected using a scanning mode.

In an aspect of the application, the mass-to-charge ratio is determined using a quadrupole analyzer. For example, in a "quadrupole" or "quadrupole ion trap" instrument, ions in an oscillating radio frequency field experience a force proportional to the DC potential applied between electrodes, the amplitude of the RF signal, and m/z. The voltage and amplitude can be selected so that only ions having a particular m/z travel the length of the quadrupole, while all other ions are deflected. Thus, quadrupole instruments can act as both a "mass filter" and as a "mass detector" for the ions injected into the instrument.

"Tandem mass spectrometry" or "MS/MS" is employed to enhance the resolution of the MS technique. In tandem mass spectrometry, a parent ion generated from a molecule of interest may be filtered in an MS instrument, and the parent ion subsequently fragmented to yield one or more daughter ions that are then analyzed (detected and/or quantified) in a second MS procedure.

Collision-induced dissociation ("CID") is often used to generate the daughter ions for further detection. In CID, parent ions gain energy through collisions with an inert gas, such as argon, and subsequently fragmented by a process referred to as "unimolecular decomposition." Sufficient energy must be deposited in the parent ion so that certain bonds within the ion can be broken due to increased vibrational energy.

By careful selection of parent ions using the first MS procedure, only ions produced by certain analytes of interest are passed to the fragmentation chamber to generate the daughter ions. Because both the parent and daughter ions are produced in a reproducible fashion under a given set of ionization/fragmentation conditions, the MS/MS technique can provide an extremely powerful analytical tool. For example, the combination of filtration/fragmentation can be used to eliminate interfering substances, and can be particularly useful in complex samples, such as biological samples.

The mass spectrometer typically provides the user with an ion scan; that is, the relative abundance of each m/z over a given range (*e.g.*, 10 to 1200 amu). The results of an analyte assay, that is, a mass spectrum, can be related to the amount of the analyte in the original sample by numerous methods known in the art. For example, given that sampling and analysis parameters are carefully controlled, the relative abundance of a given ion can be compared to a table that converts that relative abundance to an absolute amount of the original molecule.

The skilled artisan will understand that the choice of ionization method can be determined based on the analyte to be measured, type of sample, the type of detector, the choice of positive versus negative mode, etc. Ions can be produced using a variety of methods including, but not limited to, electron ionization, chemical ionization, fast atom bombardment, field desorption, and matrix-assisted laser desorption ionization (MALDI), surface enhanced laser desorption ionization (SELDI), photon ionization, electrospray ionization, and inductively coupled plasma. Electrospray ionization is a preferred ionization method. The term "electrospray ionization," or "ESI," as used herein refers to methods in which a solution is passed along a short length of capillary tube, to the end of which is applied a high positive or negative electric potential. Solution reaching the end of the tube is vaporized (nebulized) into a jet or spray of very small droplets of solution in solvent vapor. This mist of droplets flows through an evaporation chamber, which is heated to prevent condensation and to evaporate solvent. As the droplets get smaller the electrical surface charge density increases until such time that, the natural repulsion between like charges causes ions as well as neutral molecules to be released.

In some aspects, the effluent of the LC is injected directly and automatically *(i.e.,* "in-line") into the electrospray device. In certain embodiments, the at least one OxFA contained in the LC effluent is first ionized by electrospray into a parent ion. The first quadropole of the MS/MS is tuned to be a mass filter for the OxFA parent ion (and/or the internal standard).

Parent ion(s) passing the first quadropole are then ionized and/or fragmented prior to passing into the second quadropole. In certain embodiments, the ions are collided with an inert gas molecule in a process of collision-induced dissociation (CID). Examples of inert gases include, for example, argon, helium, nitrogen, etc. In some embodiments, the OxFA parent ion is fragmented into daughter ions. It is these daughter ions that are subsequently detected.

One or more standards can be employed for calibration and quantification purposes. Internal and external standards are commonly used for these purposes. Internal standards are typically analogs of the compound(s) of interest that are expected to react similarly during all extraction and quantification steps. A known amount of an internal standard is typically added to each sample early in the processing in order to account for any loss of compound during any processing step. In an exemplary embodiment, 15-HETE-d₈ (CAYMAN CHEMICALS, Ann Arbor, MI) is used as an internal standard. External standards typically consist of samples containing a known quantity of the compound of interest, or an analog, which are processed in parallel with the experimental samples. External standards are often used to control for the efficiency of the various processing steps. Finally, calibration standards are used to quantify the amount of the compound of interest in each experimental and external control sample. Typically, a series of calibration standards containing varying known amounts of the compound(s) of interest are injected directly into the detection device *(i.e.,* the MS). Calibration standards are used to generate a standard curve, against which the experimental samples are quantified. These standards also may be used to determine limits of detection for any particular detection methodology.

In some aspects, the level of at least one OxFA and/or at least one corresponding precursor molecule in a sample can be quantified using liquid chromatography online electrospray ionization tandem mass spectrometry (LC/ESI/MS/MS). In an exemplary embodiment, 50µl of plasma is hydrolyzed at 60° under argon atmosphere in the presence of potassium hydroxide for 2 hr and then the released fatty acids are extracted into the hexane layer twice by liquid/liquid extraction. The combined hexane layers are dried under nitrogen gas flow and then re-suspended in 200 µl 85% methanol/water. An aliquot of the lipid extract is then injected onto an HPLC (*e.g.*, Waters 2690 Separations Module, Franklin MA) and then OxFAs and their precursors are separated through a C18 column (Phenomenex ODS(2), 2 x 150mm, 5µm, Rancho Palos Verdes, CA) using a gradient starting from 85% methanol containing 0.2% acetic acid over 10 min and then to 100% methanol containing 0.2% acetic acid for 15 min. The OxFAs and their precursors are then quantified on a triple quadrupole mass spectrometer (*e.g.*, Quattro Ultima, Micromass., Manchester, UK) using ESI in negative ion mode and multiple reaction monitoring (MRM) using characteristic parent to daughter ion transitions for the specific molecular species monitored as described above.

Once the level or amount of the at least one OxFA and/or the ratio of at least one OxFA to a corresponding precursor molecule in a sample are determined, the level can be compared to a predetermined value or control value to provide information for diagnosing or monitoring NAFLD, NASH, and/or liver fibrosis in a subject. For example, the level of at least one OxFA in a sample can be compared to a predetermined value or control value to determine if a subject is afflicted with NAFLD, NASH, and/or liver fibrosis.

The level of at least one OxFA, in the subject's bodily sample may also be compared to the level of OxFA obtained from a bodily sample previously obtained from the subject, such as prior to administration of therapeutic. Accordingly, the method described herein can be used to measure the efficacy of a therapeutic regimen for the treatment of NAFLD, NASH, and/or liver fibrosis in a subject by comparing the level of at least one OxFA in bodily samples obtained before and after a therapeutic regimen. Additionally, the method described herein can be used to measure the progression of NAFLD, NASH, and/or liver fibrosis in a subject by comparing the level of at least one OxFA in a bodily sample obtained over a given time period, such as days, weeks, months, or years.

The level of OxFA in a sample may also be compared to a predetermined value or control value to provide information for determining the severity of the disease in the subject or the tissue of the subject (*e.g.*, liver tissue). Thus, in some aspect, a level of at least one OxFA may be compared to control values obtained from subjects with well known clinical categorizations, or stages, of histopathologies related to NAFLD and/or NASH (*e.g.*, lobular liver inflammation, liver steatosis, and liver fibrosis). In one particular embodiment, a level of at least one OxFA in a sample can provide information for determining a particular stage of fibrosis in the subject. For example, stages of fibrosis may be defined as Stage 1: no fibrosis or mild fibrosis; Stage 2: moderate fibrosis; Stage 3 and 4: severe fibrosis.

A predetermined value or control value can be based upon the level of at least one OxFA in comparable samples obtained from a healthy or normal subject or the general population or from a select population of control subjects. In some aspects, the select population of control subjects can include individuals diagnosed with NAFLD and/or NASH. For example, a subject having a greater levelor level of at least one OxFA compared to a control value may be indicative of the subject having a more advanced stage of a histopathology related to NASH.

The select population of control subjects may also include subjects afflicted with NALFD in order to distinguish subjects afflicted with NASH from those with hepatic steatosis by comparing the level of at least one OxFA in the samples. In some aspects, the select population of control subjects includes individuals afflicted with NALFD having none or minimal steatosis and none or minimal inflammation and who were classified as normal liver biopsy. In some aspects, the select population of control subjects may include a group of individuals afflicted with hepatic steatosis.

In another aspect, the select population of control subjects can include individual patients with chronic hepatitis C or alcohol liver disease in order to distinguish subjects afflicted with NASH from those with other chronic liver diseases by comparing the levels of at least one OxFA in the samples.

The predetermined value can be related to the value used to characterize the level of the OxFA in the bodily sample obtained from the test subject. Thus, if the level of the OxFA is an absolute value, such as the mass of the OxFA of the bodily sample, the predetermined value can also be based upon the mass of the OxFA in subjects in the general population or a select population of human subjects. Similarly, if the level of the OxFA is a representative value such as an arbitrary unit, the predetermined value can also be based on the representative value.

The predetermined value can take a variety of forms. The predetermined value can be a single cut-off value, such as a median or mean. The predetermined value can be established based upon comparative groups such as where the level of the OxFA in one defined group is double the level of the OxFA in another defined group. The predetermined value can be a range, for example, where the general subject population is divided equally (or unequally) into groups, or into quadrants, the lowest quadrant being subjects with the lowest amounts of the OxFA, the highest quadrant being individuals with the highest amounts of the OxFA. In an exemplary embodiment, two cutoff values are selected to minimize the rate of false positive and negative results.

Predetermined values of the OxFA, such as for example, mean levels, median levels, or "cut-off' levels, are established by assaying a large sample of subjects in the general population or the select population and using a statistical model such as the predictive value method for selecting a positivity criterion or receiver operator characteristic curve that defines optimum specificity (highest true negative rate) and sensitivity (highest true positive rate) as described in Knapp, R. G., and Miller, M. C. (1992). Clinical Epidemiology and Biostatistics. William and Wilkins, Harual Publishing Co. Malvern, Pa., which is specifically incorporated herein by reference. A "cutoff" value can be determined for each OxFA that is assayed.

Another aspect of the application relates to a method for generating a result useful in diagnosing and monitoring NAFLD, NASH, and/or liver fibrosis by obtaining a dataset associated with a sample, where the dataset includes quantitative data (typically oxidized fatty acid product levels) about oxidized fatty acid products about which have been found to be predictive of severity of NASH and/or liver fibrosis with a statistical significance less than 0.2 *(e.g.,* p value less than about 0.05), and inputting the dataset into an analytical process that uses the dataset to generate a result useful in diagnosing and monitoring NAFLD, NASH, and/or liver fibrosis. In certain embodiments, the dataset also includes quantitative data about other clinical indicia or other marker associated with NAFLD.

Datasets containing quantitative data, typically oxidized fatty acid product levels, for the various oxidized fatty acid product markers used herein, and quantitative data for other dataset components can be inputted into an analytical process and used to generate a result. The analytical process may be any type of learning algorithm with defined parameters, or in other words, a predictive model. Predictive models can be developed for a variety of NAFLD classifications by applying learning algorithms to the appropriate type of reference or control data. The result of the analytical process/predictive model can be used by an appropriate individual to take the appropriate course of action.

In an aspect of the application, a scoring system or risk score can be generated by the analytical process to diagnose and monitor NAFLD, NASH, and/or liver fibrosis. In some aspects, the analytical process can use a dataset that includes level of at least one OxFA in a subject's sample as determined by the methods described herein. The risk score can then be compared to a control value, to provide information for diagnosing NASH and/or liver fibrosis in a subject.

In other aspects, the analytical process can use a dataset that includes the determined level of the at least one oxidized fatty acid product and quantitative data from one or more clinical indicia to generate a risk score. The risk score can be derived using an algorithm that weights a level of the at least one oxidized fatty acid product in the sample and one more clinical indicia (or anthropometric features or measures) including but not limited to, age, gender, race, h/o diabetes, h/o hypertension, h/o hyperlipidemia, BMI, weight, height, waist circumference, hip/waste ratio, and other laboratory data including but not limited to aspartate aminotransferase (AST), alanine aminotransferase (ALT), AST/ALT ratio, gammaGT, bilirubin, alkaline phosphatase, albumin, prothrombin time, platelet count, creatinine, total cholesterol, HDL, LDL, Triglycerides, triglyceride:HDL ratio, fasting glucose, fasting insulin, glucose/insulin ratio and Homeostatic Model Assessment index measuring insulin resistance.

In one example, the one or more clinical indicia can include at least one of the subject's age, body mass index, or concentration of aspartate transaminase or alanine transaminase. In another example, the one or more clinical indicia can include at least two of the subject's age, body mass index, and concentration of aspartate transaminase or alanine transaminase. In still another example, the dataset can include the determined level of the at least one oxidized fatty acid product, the subject's age, body mass index, and concentration of aspartate transaminase or alanine transaminase.

In some embodiments, the oxidized fatty acid product can be a linoleic or conjugated linoleic oxidation product. In another aspect, the at least one oxidized fatty acid product can be selected from the group consisting of 12-HETE, 15-HETE, 11-HETE, 8-HETE, 9-HETE, 5-HETE, 13-HODE, 9-HODE, 9-oxoODE, 13-oxoODE, and F2isoprostanes. In a further aspect, the at least one oxidized fatty acid product can be selected from the group consisting of 13-HODE, 9-HODE, 9-oxoODE and 13-oxoODE.

In certain embodiments, an OxFA/precursor ratio can be measured and can include, for example, the ratio of at least one linoleic or conjugated linoleic oxidation product to a respective precursor molecule(s). In another aspect of the invention, an OxFA/precursor ratio can be measured and can include, for example, the ratio of at least one oxidized fatty acid product selected from the group consisting of 13-HODE, 9-HODE, 9-oxoODE and 13-oxoODE to a respective precursor molecule(s) (*e.g*., linoleic acid or conjugated linoleic acid). In other embodiments, the level of F2isoprostanes can be measured and a ratio of the F2isoprostanes to OxFA precursor can be determined.

The analytical process used to generate a risk score may be any type of process capable of providing a result useful for classifying a sample, for example, comparison of the obtained dataset with a reference dataset, a linear algorithm, a quadratic algorithm, a decision tree algorithm, or a voting algorithm.

Prior to input into the analytical process, the data in each dataset can be collected by measuring the values for each marker, usually in triplicate or in multiple triplicates. The data may be manipulated, for example, raw data may be transformed using standard curves, and the average of triplicate measurements used to calculate the average and standard deviation for each patient. These values may be transformed before being used in the models, e.g. log-transformed or Box-Cox transformed. This data can then be input into the analytical process with defined parameters.

The analytical process may set a threshold for determining the probability that a sample belongs to a given class. The probability preferably is at least 50%, at least 60%, at least 70%, at least 80%, at least 90%, or higher.

In other embodiments, the analytical process determines whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

In general, the analytical process will be in the form of a model generated by a statistical analytical method. In some embodiments, the analytical process is based on a regression model, preferably a logistic regression model. Such a regression model includes a coefficient for each of the markers in a selected set of markers disclosed herein. In such embodiments, the coefficients for the regression model are computed using, for example, a maximum likelihood approach. In particular embodiments, molecular marker data from the two groups (*e.g.*, healthy and diseased) is used and the dependent variable is the status of the patient for which marker characteristic data are from.

By way of example, the analytical process can include logistic regression model that generates a risk score based on the following algorithm: risk score = [-10.051 + 0.0463*Age(years) + 0.147*BMI(kg/m²) + 0.0293*AST(IU/L) + 2.658*13-HODE:LA Ratio(mmol/mol)] * 10) is determined. The risk score can be converted to a probability distribution with a value of 0 to 100 by the following algorithm;
oxNASH = 100*exp(z)/[1 + exp(z)], wherein oxNASH is the probability distribution and z is the risk score calculated using the above noted algorithm.

It will be appreciated, that other analytical processes can be used to generate a risk score. These analytical processes can include for example a Linear Discriminant Analysis model, a support vector machine classification algorithm, a recursive feature elimination model, a prediction analysis of microarray model, a classification and regression tree (CART) algorithm, a FlexTree algorithm, a random forest algorithm, a multiple additive regression tree (MART) algorithm, or Machine Learning algorithms.

A risk score or result generated by the analytical process can be any type of information useful for making a NAFLD classification, *e.g*., a classification, a continuous variable, or a vector. For example, the value of a continuous variable or vector may be used to determine the likelihood that a sample is associated with a particular classification.

NAFLD classification refer to any type of information or the generation of any type of information associated with NAFLD, NASH, and/or liver fibrosis, for example, diagnosis, staging, assessing extent of NAFLD, NASH, and/or liver fibrosis progression, prognosis, monitoring, therapeutic response to treatments, screening to identify compounds that act via similar mechanisms as known NAFLD, NASH, and/or liver fibrosis treatments.

In some aspects, the result is used for diagnosis or detection of the occurrence of NASH. In this embodiment, a reference or training set containing "healthy" and "NASH" samples is used to develop a predictive model. A dataset, preferably containing oxidized fatty acid product levels indicative of NASH, is then inputted into the predictive model in order to generate a result. The result may classify the sample as either "healthy" or "NASH" or staging of "NASH". In other embodiments, the result is a continuous variable providing information useful for classifying the sample, *e.g.,* where a high value indicates a high probability of being a "NASH" sample and a low value indicates a low probability of being a "healthy" sample.

In other embodiments, the result is used for NAFLD, NASH, and/or liver fibrosis staging. In this embodiment, a reference or training dataset containing samples from individuals with disease at different stages is used to develop a predictive model. The model may be a simple comparison of an individual dataset against one or more datasets obtained from disease samples of known stage or a more complex multivariate classification model. In certain embodiments, inputting a dataset into the model will generate a result classifying the sample from which the dataset is generated as being at a specified NAFLD, NASH, and/or liver fibrosis disease stage. Similar methods may be used to provide NAFLD, NASH, and/or liver fibrosis prognosis, except that the reference or training set will include data obtained from individuals who develop disease and those who fail to develop disease at a later time.

In other embodiments, the result is used determine response to NAFLD, NASH, and/or liver fibrosis treatments. In this embodiment, the reference or training dataset and the predictive model is the same as that used to diagnose NAFLD, NASH, and/or liver fibrosis (samples of from individuals with disease and those without). However, instead of inputting a dataset composed of samples from individuals with an unknown diagnosis, the dataset is composed of individuals with known disease which have been administered a particular treatment and it is determined whether the samples trend toward or lie within a normal, healthy classification versus an NAFLD, NASH, and/or liver fibrosis classification.

In another embodiment, the result is used for drug screening, *i.e.,* identifying compounds that act via similar mechanisms as known NAFLD, NASH, and/or liver fibrosis drug treatments. In this embodiment, a reference or training set containing individuals treated with a known NAFLD, NASH, and/or liver fibrosis drug treatment and those not treated with the particular treatment can be used develop a predictive model. A dataset from individuals treated with a compound with an unknown mechanism is input into the model. If the result indicates that the sample can be classified as coming from a subject dosed with a known NAFLD, NASH, and/or liver fibrosis drug treatment, then the new compound is likely to act via the same mechanism.

Using methods described herein, skilled physicians may select and prescribe treatments adapted to each individual subject based on the diagnosis of NAFLD, NASH, and/or liver fibrosis provided to the subject through determination of the levelof at least one OxFA in a subject's sample. In particular, the present invention provides physicians with a non-subjective means to diagnose NAFLD, NASH, and/or liver fibrosis, which will allow for early treatment, when intervention is likely to have its greatest effect. Selection of an appropriate therapeutic regimen for a given patient may be made based solely on the diagnosis provided by the inventive methods. Alternatively, the physician may also consider other clinical or pathological parameters used in existing methods to diagnose NAFLD, NASH, and/or liver fibrosis and assess its advancement.

Effective dosages and administration regimens can be readily determined by good medical practice and the clinical condition of the individual subject. The frequency of administration will depend on the pharmacokinetic parameters of the active ingredient(s) and the route of administration. The optimal pharmaceutical formulation can be determined depending upon the route of administration and desired dosage. Such formulations may influence the physical state, stability, rate of in vivo release, and rate of *in vivo* clearance of the administered compounds.

Depending on the route of administration, a suitable dose may be calculated according to body weight, body surface area, or organ size. Optimization of the appropriate dosage can readily be made by those skilled in the art in light of pharmacokinetic data observed in human clinical trials. The final dosage regimen will be determined by the attending physician, considering various factors which modify the action of drugs, e.g., the drug's specific activity, the severity of the damage and the responsiveness of the patient, the age, condition, body weight, sex and diet of the patient, the severity of any present infection, time of administration and other clinical factors.

One of skill will also recognize that the results generated using these methods can be used in conjunction with any number of the various other methods known to those of skill in the art for diagnosing and monitoring NAFLD, NASH, and/or liver fibrosis.

The Examples that follow illustrate embodiments of the present invention and are not limiting of the specification and claims in any way.

### EXAMPLES

### EXAMPLE 1

In this example we explore a highly sensitive liquid chromatography-mass spectrometric approach to define the circulating profile of bioactive lipid peroxidation products characteristic of patients with NASH and the role of free radical mediated processes in generation of these products. We also unexpectedly found that circulating levels of a subset of structurally specific oxidized fatty acids (oxFA's) serve as markers of hepatic inflammation, steatosis and fibrosis in NASH patients.

### Methods

### Patient characteristics and sample collection

Our cohort consisted of an initial group of 73 consecutive patients and a subsequent validation group of 49 consecutive patients in whom fasting blood was drawn the morning of scheduled elective liver biopsy at the Cleveland Clinic. The inclusion / exclusion criteria are detailed below (see Clinical Diagnosis). Extensive demographic, clinical and laboratory data were collected from each patient. Whole blood was collected into ethylenediaminetetraacetic acid (EDTA) tubes. Blood was immediately placed on ice or within a refrigerator, and samples centrifuged at 3500 rpm for 10 minutes at 4°C within 2 hr of collection. Plasma was then immediately stored under conditions to minimize artificial oxidation (*i.e.,* with antioxidant cocktail under inert atmosphere). Briefly, plasma aliquots were placed into cryotubes with screw caps and o-rings. Antioxidant cocktail (from 100 x stocks) was added consisting of butylated hydroxytoluene (500 µM final) and diethylenetriamine pentaacetic acid (2mM final). Headspace in the cryotube was purged with argon, and then sealed tubes were snap frozen in liquid nitrogen for storage at -80°C until analysis. Liver biopsy tissue was collected and sent to the Department of Anatomic Pathology for histopathological analysis (see Liver Biopsy).

### Clinical Diagnosis

Information regarding demographics, medical history, and medications were obtained by patient interview and confirmed by chart review. Race information was based on self report and the information used for analyses was pre-specified prior to the study. The clinical outcome data were verified by source documentation. Subjects included in the study were between the age of 18-70 years inclusive, had < 20 grams/day of alcohol consumption for males and <10 grams/day for females, and were referred by treating physician for a baseline liver biopsy in the context of diagnostic evaluation for suspected NAFLD. Currently there are no established guidelines of when to perform a liver biopsy in either adult or pediatric patients with suspected NAFLD. Thus, the decision to perform a baseline liver biopsy in our patient population was made on an individual basis by the treating gastroenterologist independent of the present study. In the most cases the decision for biopsy indication was based on the presence of persistently abnormal liver enzymes (mainly serum ALT) in a patient with suspected NAFLD. Patients were excluded from the study if other liver diseases were detected, including viral, drug related, autoimmune, metabolic/genetic liver disease. These other liver diseases were ruled out in all cases based on standard clinical studies, imaging and/or liver biopsy features, or laboratory studies including viral hepatitis panel, ceruloplasmin, alpha-1-antitrypsin, autoantibodies, metabolic/inborn error panel.

### Liver biopsy

Liver histology was assessed by an experienced liver pathologist blinded to patient clinical and laboratory data. Biopsy length and the average number of portal tracts were recorded for each patient. The diagnosis of NASH was established based on Brunt's Criteria. The NAFLD activity scoring system developed by the NIDDK NASH Clinical Research Network (NASH CRN) was also calculated for each patient. According to this scoring system, the degree of steatosis, liver injury and inflammatory activity are measured using a 0 to 8 scale (steatosis: 0-3; lobular inflammation: 0-3 and ballooning: 0-2). The NAFLD activity score (NAS) is the unweighted sum of steatosis, lobular inflammation and hepatocellular ballooning score. The degree of fibrosis was measured using a 6-point scale (1a, b = zone 3 perisinusoidal fibrosis; 1c = portal fibrosis only; 2 = zone 3 and portal /periportal fibrosis; 3 = bridging fibrosis; 4= cirrhosis). Severity of fibrosis was defined as: Stage 1: no fibrosis or mild fibrosis; Stage 2: moderate fibrosis; Stage 3 and 4: severe fibrosis. The quality of liver biopsies was rated as follows: Optimal quality: biopsy >2.5 cm length, more than 10 portal tracts and no fragmentation; Good quality: biopsy >1.5 cm length, more than 6 portal tracts and no fragmentation; Inadequate biopsy: <1.5 cm length, fewer than 6 portal tracts and fragmented. Only optimal and good quality samples were included in the study.

### Lipid extraction from human plasma

Lipid extractions and protein hydrolyses were performed using disposable threaded borosilicate glass test tubes with PTFE lined caps. Before use, all glassware tubes, caps and pipette tips were washed with nitric acid to remove trace transition metals, extensively rinsed with Chelex-treated water containing 1 µM diethylenetriamine pentaacetic acid (DTPA; pH 7.0 in H₂O), and then rinsed with pure Chelex-treated water. Plastic tips were further rinsed in methanol and air-dried prior to use. Test tubes were also baked at 500 °C overnight to remove residual potential organics. All plasma samples for analyses contained anti-oxidant cocktail (DTPA (2 mM final) and butylated hydroxytoluene (500 µM final) with head space overlaid with argon. Samples were thawed in ice/water bath immediately prior to sample handling for LC/MS/MS analysis. Fatty acids and oxidized fatty acids in plasma were extracted. Briefly, plasma (50 µl), internal standard (synthetic 15(S)-HETE-d8) and potassium hydroxide were added to the glass test tubes, overlaid with argon and sealed. Lipids were hydrolyzed at 60°C under argon atmosphere for 2 hrs and then the released fatty acids were extracted into the hexane layer twice by liquid/liquid extraction. With each extraction, argon was used to purge the head space of the tube prior to sealing and vortexing/centrifugation. The combined hexane layers were dried under nitrogen gas and then re-suspended in 200 µl 85% methanol/water (v/v).

### Liquid chromatography online electrospray ionization tandem mass spectrometry (LC/ESI/MS/MS)

Levels of multiple fatty acid oxidation products (free plus esterified) in plasma were quantified using LC/ESI/MS/MS (Fig. 1). Briefly, lipid extract was injected onto an HPLC (Waters 2690 Separations Module, Franklin MA) and the oxidized fatty acids and their precursors were separated through a C18 column (Phenomenex ODS(2), 2 x 150mm, 5 µm, Rancho Palos Verdes, CA) using a gradient starting from 85% methanol containing 0.2% acetic acid over 10 min and then to 100% methanol containing 0.2% acetic acid over 2 min, following by 100% methanol containing 0.2% acetic for 15 min. The oxidized fatty acids and their precursors were quantified on a triple quadrupole mass spectrometer (Quattro Ultima, Micromass., Manchester, UK) using ESI in negative ion mode and multiple reaction monitoring (MRM) using characteristic parent -> daughter ion transitions for the specific molecular species monitored. The lipid peroxidation products analyzed included structurally specific species of hydroxy-eicosatetraenoic acids (HETEs 5, 8, 9, 11, 12 and 15), hydroxy-octadecadienoic acids (HODEs 9 and 13), oxo-octadecadienoic acids (HODEs 9 and 13) and their precursor's arachidonic acid and linoleic acid. The sample preparation and the quantitation of oxidized fatty acids by LC/ESI/MS/MS were performed by an investigator who was blinded to the liver histology and other clinical data. 15-HETE-d₈ (Cayman Chemicals, Ann Arbor, Michigan) was used as internal standard for calibration of oxidized fatty acids in plasma. To further assess the role of free radical versus stereoselective (enzymatic) processes in formation the lipid peroxidation molecular species, isomers of 13-HODE, 13(S)-HODE and 13(R)-HODE were separated by liquid chromatography on a chiral phase to quantify stereo specificity. Lipid extract was injected onto and separated through a Chiral column (Chiralpak IA, 4.6 x 250 mm, Chiral Tech. Inc., West Chester, PA) on a HPLC (Beckman 126, Palatine, IL) using mobile phase hexane/2-propanol (90/10,v/v) at a flow rate of 0.5 ml/min. Based on the difference of their retention time versus authentic synthetic chiral standards, the HPLC fractions containing 13(S)-HODE and 13(R)-HODE were individually collected, dried under nitrogen gas flow and reconstituted in 50% methanol. The quantity of 13(S)-HODE and 13(R)-HODE were then determined by LC/ESI/MS/MS.

In control studies both the influence of sample processing time and assay methodology were evaluated to ensure no artificial formation of oxidation products occurred under the conditions employed. Linoleate-d4 and arachidonate-d8 (Cayman Chemicals, Ann Arbor, Michigan) were added to human plasma (100 µM each final) that had either been isolated immediately following blood draw, or from whole blood (drawn into a purple top EDTA plasma tube) that was kept on ice for 4 hours prior to plasma isolation. Plasma samples were then treated with the typical antioxidant cocktail of BHT and DTPA, and analyzed under typical conditions as outlined above. During LC/MS/MS analyses parent --> daughter transitions were monitored for both the endogenous fatty acids and their oxidation products, as well as the isotopomers that would be formed from the deuterated parent fatty acids if artificial oxidation occur. After completion of the plasma analyses using the above assay methods, only trace (or none for many species) levels of the deuterated HODEs, oxoODEs and HETEs were detected, with calculated production of all monitored structurally specific oxidized fatty acids being < 5% of the endogenous levels detected. Moreover, comparison of the analyses from plasma isolated immediately upon blood draw, versus delay on ice prior to plasma isolation, showed similar results (within +/- 5%). These data confirm that the sample handling prior to plasma isolation and assay methodology used did not significantly artificially produce specific lipid oxidation products monitored.

### Statistical analysis

Clinical diagnosis, histopathological diagnosis, laboratory and mass spectrometry assays were performed by investigators blinded to sample identity other than study barcode. Continuous variables are presented as median (25th, 75th percentiles) and categorical variables as numbers and percentages. Kruskal-Wallis tests for continuous and ordinal factors and Pearson's chi-square for categorical factors were used to assess differences between the three patient groups. Ad-hoc pair wise comparisons were done using Pearson's Chi-square for categorical factors and the Stee-Dwass procedure for continuous factors; a significance criterion of 0.017 was used for these. Spearman Rank correlation coefficients (rho) were calculated to test the correlation between the different oxidized fatty acid levels with liver histology features (inflammation, degree of steatosis and degree of fibrosis) and the HOMA index for metabolic function. A multivariable logistic regression model for prediction of NASH in the initial cohort was constructed by performing automated stepwise variable selection on 1,000 bootstrap samples; the 4 most frequently included variables were incorporated in the final model. The multivariable logistic regression model (z) obtained from the original cohort was defined as follows: z = -10.051 + 0.0463*Age(years) + 0.147*BMI(kg/m²) + 0.0293*AST(IU/L) + 2.658*HODE-13:LA Ratio(mmol/mol)]. This was then converted into a probability distribution with value between 0 to 100 and called "oxNASH" by the following algorithm: oxNASH= 100*exp(z)/[1 + exp(z)]. Using this algorithm, the oxNASH was then calculated for an independent group of patients in order to validate the predictive ability of the score. The area under the Receiver Operating Characteristic (ROC) curves for AST, ALT and the final model were estimated and compared using Delong's method. Subgroup analyses were carried out to compare 13-HODE, 9-HODE and 9-oxoODE levels according to the presence or absence of metabolic factors (diabetes mellitus, hypertension and obesity) in patients with histopathological diagnosis of NASH. A P<0.05 was considered statistically significant. SAS version 9.2 software (The SAS Institute, Cary, NC) and R version 2.4.1 software (The R Foundation for Statistical Computing, Vienna, Austria) were used for all analyses.

### Results

### Systemic levels of a select subset of oxFA are markedly increased in plasma of patients with NASH.

We initially quantified the oxFA profile using LC/MS/MS in a well characterized group of patients with suspected NAFLD. The main clinical and serological features of the study participants stratified according to their liver biopsy results are summarized in Table 1. Of the initial 73 subjects (initial cohort), 37 had NASH (51%); 23 had hepatic steatosis (32%) and 13 subjects had either none or minimal steatosis (< than 5%) and none or minimal inflammation and were classified as "controls" (*i.e.,* normal liver biopsy, 17%). Participants were of similar age, and predominantly Caucasian. Subjects with NASH had greater prevalence of history of diabetes mellitus, hyperlipidemia, and hypertension but this did not reach statistical significance. The level of high density lipoprotein cholesterol (HDL) was lower in participants with NASH while triglyceride levels were significantly higher in the NASH group. Patients with NASH had significantly higher body mass index (BMI) and the Homeostatic Model Assessment index (HOMA), which is a sensitive measure of insulin resistance.

**Table 1**

| **Factor** | **Normal Bx (N=13)** | **Steatosis (N=23)** | **NASH (N=37)** | **p-value** |
|---|---|---|---|---|
| ***Demographic*** | | | | |
| Female | 7 (53.9) | 11 (47.8) | 21 (56.8) | 0.8 |
| Caucasian | 10 (76.9) | 18 (78.3) | 33 (89.2) | 0.42 |
| Age | 45 (42, 53) | 47 (38, 59) | 53 (44, 58) | 0.26 |

| ***Clinical*** | | | | |
|---|---|---|---|---|
| BMI | 29.7 (26.8, 32) | 30.6 (27, 33.4) | 32 (30.7, 34.5) | **0.039** |

| **Factor** | **Normal Bx (N=13)** | **Steatosis (N=23)** | **NASH (N=37)** | **p-value** |
|---|---|---|---|---|
| AST | 43 (34, 56) | 50 (36, 60) | 63 (46, 84)^{a} | **0.019** |
| ALT | 59 (44, 71) | 65 (42, 90) | 82 (42, 112) | 0.56 |
| HOMA | 1 (0.4, 1.6) | 1.4 (0.8, 2.7) | 5.5 (3, 12.9)^{a,b} | **<0.001** |
| Diabetes | 3 (25) | 5 (21.7) | 14 (38.9) | 0.34 |
| Hyperlipidemia | 7(58.3) | 10(43.5) | 20 (55.6) | 0.59 |
| HTN | 3 (25) | 8 (36.4) | 18(51.4) | 0.22 |
| Platelet | 269 (231.5, 304.5) | 247 (207, 312) | 225 (199, 262) | 0.076 |
| Cholesterol | 160(158,211) | 211 (169, 239) | 192 (162, 236) | 0.39 |
| Triglycerides | 86 (69, 94) | 160 (107, 210)^{a} | 192 (139, 241)^{a} | **0.003** |
| HDL | 62 (50, 70) | 56 (47, 63) | 44 (40, 50)^{a,b} | **0.003** |
| LDL | 77.6 (75.2, 128) | 119.0 (94.4, 135.6) | 107.0 (94.4, 153.4) | 0.54 |

| ***Histologic*** | | | | |
|---|---|---|---|---|
| Fibrosis | | | | **<0.001** |
| 0 | 13 (100) | 20 (87) | 2 (5.4)^{a,b} | |
| 1 | 0 (0.0) | 3 (13.0) | 11 (29.7) | |
| 2 | 0 (0.0) | 0 (0.0) | 6(16.2) | |
| 3 | 0 (0.0) | 0 (0.0) | 12 (32.4) | |
| 4 | 0 (0.0) | 0 (0.0) | 6 (16.2) | |
| Inflammation | | | | ---- |
| None/minimal | 13 (100) | 19(82.6) | 0 (0.0) | |
| Mild | 0 (0.0) | 4(17.4) | 16(44.4) | |
| Moderate | 0(0.0) | 0(0.0) | 19(52.8) | |
| Severe | 0(0.0) | 0(0.0) | 1 (2.8) | |
| Steatosis | | | | ---- |
| None/minimal | 13 (100) | 0 (0.0) | 0 (0.0) | |
| 5-33% | 1(7.7) | 13 (56.5) | 4(11.1) | |
| 34-66% | 0(0.0) | 10 (43.5) | 20 (55.6) | |
| >66% | 0(0.0) | 0(0.0) | 12 (33.3) | |
| Ballooning | | | | ---- |
| None | 13 (100) | 21 (91.3) | 1 (2.8) | |
| Few | 0 (0.0) | 2(8.7) | 16(44.4) | |
| Many | 0(0.0) | 0(0.0) | 19(52.8) | |
| NAS | 0.0 (0.0, 0.0) | 2(1,2) | 5 (5, 6) | ---- |

| | | | | |
|---|---|---|---|---|
| Values presented as Mean (± SD); or Median (25^{th} -75^{th} quartile); a: Significantly different from Normal biopsy group; b: Significantly different from Hepatic Steatosis Group. Pairwise comparisons where done using Pearson's chi-square for categorical factors and the Stee-Dwass procedure for continuous factors. Significance criterion is 0.017. Diabetes mellitus: history of (h/o) diabetes, fasting plasma glucose ≥126mg/dL and/ or on hypoglycemic agents, Hypertension: h/o hypertension, systolic and diastolic blood pressure ≥ 120/80 mmHg and/or on anti-hypertensive agents, Hyperlipidemia: h/o high plasma cholesterol, total cholesterol ≥200 mg/dL and /or on hypolipidemic agents; BMI: body mass index; ALT: Alanine Transaminase; AST: Aspartate Transaminase; HOMA index: Homeostatic Model Assessment index; mg/dL: milligrams per deciliter; IU/L: international unit per liter. | | | | |

Plasma levels of multiple structurally specified oxFA were quantified in our study participants and the levels were analyzed for their relationship with histopathological changes. Remarkably, of the markers monitored, 9- and 13-HODEs and 9- and 13-oxoODEs, products of free radical-mediated oxidation of linoleic acid, were significantly elevated in patients with NASH compared to patients with hepatic steatosis and normal liver biopsy (Fig. 2). Patients with simple steatosis had slightly higher indices of oxidative stress as compared to patients on the normal liver biopsy group, but the difference in oxFA levels between the two groups failed to reach statistical significance for most analytes monitored (Fig. 2). The data expressed as the ratio of oxFA product to specific precursor were consistent with these findings and showed that patients with NASH had the highest oxlipid / precursor ratio for 9- and 13-HODEs and 9- and 13-oxoODEs as compared to patients with hepatic steatosis and normal liver biopsy (Table 2). The lipid oxidation product / precursor ratio was similar in the hepatic steatosis and normal liver biopsy group of subjects for most of the fatty acid oxidation products monitored.

**Table 2**

| **Plasma levels of oxidized Fatty Acid:Precursor Ratios in patients with suspected NAFLD** | | | | |
|---|---|---|---|---|
| **Lipid:Precursor (mmol/mol)** | **Normal biopsy (N = 13)** | **Steatosis (N = 20)** | **NASH (N = 37)** | **p-value** |
| **12-HETE** | 0.14 (0.09, 0.33) | 0.24 (0.11, 0.32) | 0.25 (0.16, 0.39) | 0.17 |
| **15-HETE** | 0.54 (0.22, 1.09) | 0.57 (0.30, 0.99) | 0.86 (0.48, 1.09) | 0.3 |
| **11-HETE** | 0.22 (0.09, 0.40) | 0.25 (0.14, 0.32) | 0.27 (0.19, 0.41) | 0.31 |
| **8-HETE** | 0.24 (0.07, 0.27) | 0.24 (0.12, 0.30) | 0.25 (0.18, 0.35) | 0.21 |
| **9-HETE** | 0.33 (0.06, 0.54) | 0.30 (0.14, 0.43) | 0.37 (0.28, 0.60) | 0.16 |
| **5-HETE** | 0.37 (0.11, 0.64) | 0.45 (0.24, 0.68) | 0.51 (0.34, 0.72) | 0.21 |
| **13-HODE** | 0.29 (0.22, 0.47) | 0.31 (0.21, 0.57) | 0.51 (0.35, 0.77) | **0.01** |
| **9-HODE** | 0.43 (0.30, 0.74) | 0.44 (0.28, 0.89) | 0.72 (0.46, 1.08) | **0.02** |
| **9-oxoODE** | 0.10 (0.08, 0.16) | 0.13 (0.09, 0.19) | 0.24 (0.16, 0.32) | **0.002** |

| | | | | |
|---|---|---|---|---|
| Lipid:precursor ratio: ratio of HETE(s), HODE(s), and oxoODE(s) to their parent compound arachidonic acid and linoleic acid, respectively; Median (25^{th}-75^{th} percentile) reported for all parameters. Abbreviations: HETE: hydroxy-eicosatetraenoic acid; HODE: hydroxy-octadecadienoic acid; oxoODE: oxo-octadecadienoic acid; mmol/mol: milimols per mol of plasma. | | | | |

### OxFA levels correlate with liver histopathology independent of other metabolic factors.

A strong positive correlation was revealed between systemic levels of specific oxidation products with liver histology that included inflammation, degree of steatosis and stage of fibrosis (Table 3). The Spearman Rank correlation (r) between 13-HODE with inflammation were 0.42 (p<0.001). A similar positive correlation was observed between 13-HODE and both degree of steatosis and stage of fibrosis (r = 0.36, p<0.01; and r = 0.36, p = 0.01, respectively). We also compared circulating oxFA levels in the subgroup of NASH patients according to the presence or absence of diabetes mellitus, hypertension and obesity. Of the 37 patients (initial cohort) with NASH, 14 (38.9%) had diabetes, 18 (51.4%) hypertension and 29 (78.4%) were obese. Levels of 13-HODE, 9-HODE and 9-oxoODE levels were not found to be significantly associated with either factor (Table 3).

**Table 3: Oxidized Fatty Acids and Disease Severity**

| **Lipids** | **Inflammation** | **Steatosis** | **Fibrosis** | **NAS** |
|---|---|---|---|---|
| | **Spearman's correlation coefficients (rho)** | | | |
| 12-HETE | 0.096 | 0.131 | 0.121 | 0.137 |
| 15-HETE | 0.136 | 0.127 | 0.118 | 0.16 |
| 11-HETE | 0.085 | 0.082 | 0.081 | 0.119 |
| 8-HETE | 0.126 | 0.109 | 0.11 | 0.137 |
| 9-HETE | 0.098 | 0.186 | 0.111 | 0.185 |
| 5-HETE | 0.106 | 0.125 | 0.073 | 0.17 |
| AA | -0.169 | -0.211 | -0.18 | -0.214 |
| 13-HODE | 0.415*** | 0.355** | 0.359** | 0.418*** |
| 9-HODE | 0.387*** | 0.338** | 0.299* | 0.399*** |
| 9-oxoODE | 0.438*** | 0.440*** | 0.300* | 0.499*** |
| 13-oxoODE | 0.362** | 0.396*** | 0.247* | 0.425*** |
| LA | 0.095 | 0.031 | 0.114 | 0.059 |

| | | | | |
|---|---|---|---|---|
| p-values* p<0.05; **p<0.01; ***p<0.001; Abbreviations: HETE: hydroxy-eicosatetraenoic acid; HODE: hydroxy-octadecadienoic acid; oxoODE: oxo-octadecadienoic acid; AA, arachidonic acid; LA, linoleic acid. | | | | |

**Table 4: Metabolic Factors and Oxidized Fatty Acids in Patients with NASH**

| ***Ox-FA (pmol*/*mL )*** | **Diabetes (N=14)** | **No Diabetes (N=22)** | **P-value** | **HTN (N=18)** | **No HTN (N=17)** | **P-value** | **Obese (N=29)** | **Non-Obese (N=8)** | **P-value** |
|---|---|---|---|---|---|---|---|---|---|
| 13-HODE | 175 (119, 255) | 151 (92, 236) | 0.3 | 180 (119, 255) | 159 (111, 188) | 0.5 | 162 (115, 236) | 151 (100, 239) | 0.7 |
| 9-HODE | 255 (206, 338) | 176(146, 336) | 0.3 | 257 (165, 429) | 244 (162, 304) | 0.4 | 247 (163, 336) | 175 (142, 349) | 0.6 |
| 9-oxoODE | 77 (50, 93) | 74 (44, 105) | 1.0 | 80 (50, 105) | 75 (47, 93) | 0.5 | 76 (50, 104) | 47 (40, 85) | 0.2 |
| 13-oxoODE | 54 (35, 71) | 47 (34, 63) | 0.5 | 54 (35, 69) | 47 (30, 57) | 0.3 | 48 (35, 64) | 43 (36, 58) | 0.7 |

### OxFA level profile for NASH diagnosis

To ascertain whether plasma oxFA levels independently predicted the presence of NASH, we conducted multivariable logistic regression analysis. A result for the histopathologic diagnosis of NASH called "oxNASH" was generated by multivariable modeling that showed the best prediction for NASH diagnosis. oxNASH was calculated from the ratio of 13-HODE to linoleic acid (LA), age, BMI and AST (Fig. 3). In this prediction model a 0.5 mmol/mol increase in 13-HODE/LA ratio was associated with a 3.8-fold increase in the likelihood of having NASH (OR: 3.8 (95% CI: 1.4-10.6; p = 0.01)) (Table 5). The addition of other factors including gender, race, history of diabetes, history of hypertension, HDL, Tg:HDL ratio, history of hyperlipidemia, and HOMA did not have a confounding effect on the model. Within the initial cohort, the area under the curve (AUC) for oxNASH was estimated to be 0.83 (95% CI: 0.73, 0.93) and was found to be significantly higher than the AUCs of either that of serum ALT 0.56 (95% CI: 0.43, 0.70) or serum AST 0.69 (95% CI: 0.56, 0.81) (p<0.01) (Fig. 3A). Figure 3B illustrates how the distribution of levels of oxNASH was significantly elevated in patients with NASH compared to patients with either hepatic steatosis (P<0.01) or normal (P<0.01) liver biopsy (Fig. 3B). Using the AUC curve, two cutoff values were selected to minimize the rate of false positive and negative results. A low cutoff point for oxNASH of 55 was able to exclude the presence of NASH with a sensitivity of 81 %, and a high cutoff value for oxNASH of 73 was able to detect the presence of NASH with 97% specificity.

**Table 5: Multivariable modeling of oxidized fatty acids in prediction of NASH**

| **Factor** | **OR (95% CI)** | **p-value** |
|---|---|---|
| Age (5 year increase) | 1.3 (0.97, 1.6) | 0.083 |
| BMI (1 kg/m² increase) | 1.2 (1.02, 1.3) | 0.025 |
| AST (5 IU/L increase) | 1.2 (1.03, 1.3) | 0.01 |
| 13-HODE/LA (0.5 mmol/mol increase) | 3.8 (1.4, 10.6) | 0.011 |

| | | |
|---|---|---|
| Abbreviations: OR: odds ratio; CI: confidence interval | | |

We next examined the diagnostic accuracy of the prediction model, oxNASH, in an independent validation cohort of 49 consecutive patients. Within the validation cohort, 19 (39%) had NASH, 22 (45%) had hepatic steatosis, and 8 were classified as normal biopsies (16%). The AUC for oxNASH in the validation cohort for prediction of histopathologic diagnosis of NASH was 0.74 (95% CI: 0.6, 0.88) (Fig. 3C). By applying the low cutoff point in the validation set, the diagnosis of NASH on liver biopsy was able to be excluded with a sensitivity of 84%. While applying the high cutoff the presence of NASH was able to be established with a specificity of 63%. The risk of NASH diagnsosis for subjects with oxNASH levels in the second or third tertile versus the lowest tertile was 3.5-fold (P<0.001) and 9.7-fold (p<0.001) higher odds, respectively (Fig. 4).

### Free radical mediated processes are key mediators of lipid oxidation in NASH

The initial findings showing significantly higher levels of 9-HODE and 13-HODE in patients with NASH suggested that free radical mediated oxidation process are involved in the generation of lipid oxidation products in these patients. To further assess the role of free radical versus stereoselective (enzymatic) processes in the formation of lipid oxidation products in patients with NAFLD, chiral phase separation of individual stereoisomers coupled with mass spectrometry was used to identify and quantify the structural isomers and the chiral distribution of specific oxidized lipid species. A significant increase in peak areas of both 13(S) -HODE and 13(R)-HODE were observed in patients with NASH as compared to patients with simple steatosis, and in comparison with patients having normal liver biopsy. Furthermore, the peak area of 13(S)- HODE was similar to that of 13 (R)-HODE with a p-value of 0.1 (Fig. 5) Taken together, these observations strongly suggest that in the context of NASH, free radical mediated processes are mainly responsible for generation of lipid oxidation products (particularly 13-HODE).

### EXAMPLE 2

We developed the oxNASH score using the ratio of 13- hydroxy-octadecadienoic acid (13-HODE) to linoleic acid, age, body mass index, and aspartate transaminases. In the Example we further assessed the correlation between oxNASH and individual histologic features of NASH (steatosis, ballooning, inflammation, and fibrosis) and to assessed the correlation with the NAFLD activity score (NAS).

### Methods

Our cohort consisted of 122 patients undergoing liver biopsy for clinical suspicion of NAFLD. Liver histology was assessed by an experienced hepatopathologist blinded to clinical and laboratory data. The grade of steatosis, hepatocyte ballooning, and inflammatory activity was measured using a 0 to 8 scale (steatosis 0-3, ballooning 0-2, and lobular inflammation 0-3). NAS was calculated for each patient. The stage of fibrosis was measured using a 4-point scale. Blood was collected from each patient at the time of liver biopsy. Levels of fatty acid oxidation products were quantified using tandem mass spectrometry and OxNASH was calculated as previously described. Analysis of covariance was performed to study the association between histology and oxNASH. A p value < 0.05 was considered statistically significant.

### Results

The mean age was 49.3 (± 11.6) years and the mean BMI was 31.5 (± 4.8) kg/m². The majority of patients were Caucasian (82%) and 48% were female. OxNASH scores were significantly higher in females (64 ± 24 vs. 52 ± 30; p=0.017) and subjects with dyslipidemia (63 ± 27 vs. 52 ±27; p=0.024) or hypertension (65 ± 22 vs. 53 ± 30; p=0.013). As shown in Figs. 6-9 and Table 6 below, OxNASH correlated with NAS and its individual histologic features (steatosis, inflammation, and ballooning. *P* <0.05) with the strongest association being with inflammation [rho (95% CI) = 0.40 (0.23-0.57), p < 0.001]. Furthermore, there was a correlation between the stage of fibrosis and oxNASH (p = 0.001). These associations remained statistically significant after adjusting for multiple confounders including gender, dyslipidemia and hypertension. In adult patients with NAFLD, oxNASH correlates with histologic features of NASH especially inflammation and fibrosis and in a noninvasive marker for NASH.

**TABLE 6**

| | oxNASH T2 vs. T1 | oxNASH T3 vs. T1 |
|---|---|---|
| Fibrosis 1-4 vs. 0 | 2.06 (0.85, 5.2) | 5.08 (2.0, 13.1) |
| Fibrosis 2-4 vs. 0-1 | 1.2 (0.36, 3.8) | 4.4 (1.5, 12.9) |
| Fibrosis 3-4 vs. 0-2 | 0.61 (0.16, 2.4) | 2.08 (0.69, 6.3) |
| Steotosis 1-3 vs. 0 | 4.03 (1.3, 12.6) | 5.2 (1.5, 17.6) |
| Steotosis 2-3 vs. 0-1 | 2.5 (1.02, 6.2) | 1.9 (0.77, 4.6) |
| Inflammation 1-3 vs. 0 | 2.7 (1.06, 6.8) | 7.9 (2.9, 21.4) |
| Inflammation 2-3 vs. 0-1 | 1.7 (0.49, 5.9) | 4.4 (1.4, 13.5) |
| Ballooning 1-21 vs. 0 | 2.9 (1.1, 7.7) | 7.8 (2.8, 21.3) |
| Values presented as Odds Ratio (95% confidence interval) | | |

## Claims

1. A method of predicting, detecting, or monitoring nonalcoholic steatohepatitis in a subject with or suspected of having nonalcoholic fatty liver disease, the method comprising:
analysing a bodily sample obtained from blood, serum or plasma from the subject,
the sample including at least
one oxidized fatty acid product;
determining level of the at least one oxidized fatty acid product in the sample; and
deriving a risk score using the determined level, wherein an increased risk score compared to a control is indicative of an increased severity or risk of nonalcoholic steatohepatitis, wherein the risk score is derived using an analytical process, the analytical process using a dataset that includes the determined level of the at least one oxidized fatty acid product and quantitative data from one or more clinical indicia including at least one of the subject's age, body mass index, or concentration of aspartate transaminase or alanine transaminase.

2. The method of claim 1, the one or more clinical indicia comprising at least two of the subject's age, body mass index, and concentration of aspartate transaminase or alanine transaminase.

3. The method of claim 1, wherein dataset including the determined level of the at least one oxidized fatty acid product, the subject's age, body mass index, and concentration of aspartate transaminase or alanine transaminase.

4. The method of claim 1, wherein the analytical process comprises using a Linear Discriminant Analysis model, a support vector machine classification algorithm, a recursive feature elimination model, a prediction analysis of microarray model, a Logistic Regression model, a CART algorithm, a FlexTree algorithm, a random forest algorithm, a MART algorithm, or Machine Learning algorithms.

5. The method of claim 1, wherein said process comprises using a Linear Discriminant Analysis model or a Logistic Regression model, and said model comprises terms for the dataset selected to provide a quality metric greater than about 0.8 for an increased severity or risk of nonalcoholic steatohepatitis.

6. The method of claim 1, further comprising obtaining a plurality of risk scores for a plurality of samples obtained at a plurality of different times from the subject.

7. The method of claim 1, the statistical significance (p value) of the level of at least one oxidized fatty acid product in a subject with nonalcoholic steatohepatitis compared to a level in a normal subject being less than 0.2.

8. The method of claim 7, wherein the p value is less than about 0.05.

9. The method of claim 1, the at least one oxidized fatty acid product is selected from the group consisting of 12-HETE, 15-HETE, 11-HETE, 8-HETE, 9-HETE, 5-HETE, 13-HODE, 9-HODE, 9-oxoODE, 13-oxoODE, and F2isoprostanes.

10. The method of claim 1, the at least one oxidized fatty acid product is selected from the group consisting of 13-HODE, 9-HODE, 9-oxoODE, and 13-oxoODE.

11. The method of claim 1, further comprising comparing the ratio the at least one oxidized fatty acid product to at least one oxidized fatty acid product precursor molecule.

12. The method of claim 11, at least one oxidized fatty acid product precursor molecule comprising arachidonic acid or linoleic acid.

13. The method of claim 1, the control comprising the risk score of a normal or healthy subject or tissue.

14. The method of claim 1, wherein the analytical process for determining the risk score comprises the algorithm: risk score = [-10.051 + 0.0463*Age(years) + 0.147*BMI(kg/m²) + 0.0293*(AST or ALT)(IU/L) + 2.658*(Oxidized fatty acid product: Oxidized fatty acid precursor Ratio(mmol/mol)] * 10.

15. The method of claim 1, wherein the analytical process for determining the risk score comprises the algorithm: risk score = [-10.051 + 0.0463*Age(years) + 0.147*BMI(kg/m²) + 0.0293*AST(IU/L) + 2.658*HODE-13:LA Ratio(mmol/mol)] * 10.

16. The method of claim 15, wherein a risk score of at 2.2 indicates that the subject has or a substantially increased risk of NASH.

## Patentansprüche

1. Verfahren zum Vorhersagen, Nachweisen oder Verfolgen nichtalkoholischer Steatohepatitis bei einem Patienten mit oder mit vermuteter nichtalkoholischer Fett-Leber-Krankheit, wobei das Verfahren umfasst:
Analysieren einer Körperprobe, erhalten aus Blut,
Serum oder Plasma von dem Patienten, wobei die Probe mindestens
ein oxidiertes Fettsäure-Produkt einschließt;
Ermitteln des Spiegels des mindestens einen oxidierten Fettsäure-Produkts in der Probe; und
Ableiten einer Risiko-Einstufung unter Verwendung des ermittelten Spiegels, wobei eine erhöhte Risiko-Einstufung, verglichen mit einer Kontrolle, eine erhöhte Schwere oder ein erhöhtes Risiko einer nichtalkoholischen Steatohepatitis anzeigt, wobei die Risiko-Einstufung unter Verwendung eines analytischen Verfahrens abgeleitet wird, wobei das analytische Verfahren einen Datensatz verwendet, der den ermittelten Spiegel des mindestens einen oxidierten Fettsäure-Produkts und quantitative Daten von einem oder mehreren klinischen Anzeichen, die mindestens eines von dem Patienten-Alter, Body-Mass-Index oder der Konzentration von Aspartat-Transaminase oder Alanin-Transaminase umfassen, einschließt.

2. Verfahren nach Anspruch 1, wobei das eine oder die mehreren klinischen Anzeichen mindestens zwei von dem Patienten-Alter, Body-Mass-Index und der Konzentration von Aspartat-Transaminase oder Alanin-Transaminase umfassen.

3. Verfahren nach Anspruch 1, wobei der Datensatz den ermittelten Spiegel von dem mindestens einen oxidierten Fettsäure-Produkt, das Patienten-Alter, den Body-Mass-Index und die Konzentration von Aspartat-Transaminase oder Alanin-Transaminase einschließt.

4. Verfahren nach Anspruch 1, wobei das analytische Verfahren unter Verwendung eines linearen Diskriminanz-Analyse-Modells, eines Support-Vector-Machine-Klassifizierungs-Algorithmus, eines Recursive-Feature-Elimination-Modells, einer Vorhersage-Analyse eines Microarray-Modells, eines logistischen Regressions-Modells, eines CART-Algorithmus, eines FlexTree-Algorithmus, eines Random-Forest-Algorithmus, eines MART-Algorithmus oder maschinellen Lern-Algorithmus umfasst.

5. Verfahren nach Anspruch 1, wobei das Verfahren die Verwendung eines linearen Diskriminanz-Analyse-Modells oder eines logistischen Regressions-Modells umfasst und das Modell Terme für den Datensatz umfasst, ausgewählt, um eine Qualitäts-Metrik größer als etwa 0,8 für eine erhöhte Schwere oder ein erhöhtes Risiko von nichtalkoholischer Steatohepatitis bereitzustellen.

6. Verfahren nach Anspruch 1, weiterhin umfassend Gewinnen einer Vielzahl von Risiko-Einstufungen für eine Vielzahl von Proben, erhalten bei einer Vielzahl von verschiedenen Zeiten von dem Patienten.

7. Verfahren nach Anspruch 1, wobei die statistische Signifikanz (p-Wert) des Spiegels von mindestens einem oxidierten Fettsäure-Produkt bei einem Patienten mit nichtalkoholischer Steatohepatitis, verglichen mit einem Spiegel in einem normalen Patienten, weniger als 0,2 ist.

8. Verfahren nach Anspruch 7, wobei der p-Wert weniger als etwa 0,05 ist.

9. Verfahren nach Anspruch 1, wobei das mindestens eine oxidierte Fettsäure-Produkt aus der Gruppe, bestehend aus 12-HETE, 15-HETE, 11-HETE, 8-HETE, 9-HETE, 5-HETE, 13-HODE, 9-HODE, 9-OxoODE, 13-OxoODE und F2Isoprostanen, ausgewählt ist.

10. Verfahren nach Anspruch 1, wobei das mindestens eine oxidierte Fettsäure-Produkt aus der Gruppe, bestehend aus 13-HODE, 9-HODE, 9-OxoODE und 13-OxoODE, ausgewählt ist.

11. Verfahren nach Anspruch 1, weiterhin umfassend Vergleichen des Verhältnisses des mindestens einen oxidierten Fettsäure-Produkts mit mindestens einem oxidierten Fettsäure-Produkt-Vorstufen-Molekül.

12. Verfahren nach Anspruch 11, wobei mindestens ein oxidiertes Fettsäure-Produkt-Vorstufen-Molekül Arachidonsäure oder Linolsäure umfasst.

13. Verfahren nach Anspruch 1, wobei die Kontrolle die Risiko-Einstufung von einem normalen oder gesunden Patienten oder Gewebe umfasst.

14. Verfahren nach Anspruch 1, wobei das analytische Verfahren zur Ermittlung der Risiko-Einstufung den Algorithmus: Risiko-Einstufung = [-10,051 + 0,0463 * Alter (Jahre) + 0,147 * BMI (kg/m²) + 0, 0293 * (AST oder ALT) (IU/L) + 2,658 * (Oxidiertes Fettsäure-Produkt: oxidiertes Fettsäure-Vorstufen-Verhältnis (mMol/Mol)] * 10 umfasst.

15. Verfahren nach Anspruch 1, wobei das analytische Verfahren zur Ermittlung der Risiko-Einstufung den Algorithmus: Risiko-Einstufung = [-10,051 + 0,0463 * Alter (Jahre) + 0,147 * BMI (kg/m²) + 0,0293 * AST (IU/L) + 2,658 * HODE-13:LA-Verhältnis (mMol/Mol)] * 10 umfasst.

16. Verfahren nach Anspruch 15, wobei eine Risiko-Einstufung bei 2,2 anzeigt, dass der Patient NASH oder ein wesentlich erhöhtes Risiko dafür hat.

## Revendications

1. Procédé pour prédire, détecter ou surveiller une hépatite graisseuse non alcoolique chez un sujet ayant ou suspecté d'avoir une maladie hépatique graisseuse non alcoolique, lequel procédé comprend :
l'analyse d'un échantillon corporel obtenu à partir de sang, de sérum ou de plasma du sujet, l'échantillon contenant au moins un produit d'acide gras oxydé ;
la détermination du niveau de l'au moins un produit d'acide gras oxydé dans l'échantillon ; et
la dérivation d'un score de risque utilisant le niveau prédéterminé,
dans lequel un score de risque accru en comparaison avec un témoin est indicatif d'une gravité ou d'un risque accru d'hépatite graisseuse non alcoolique, et dans lequel le score de risque est dérivé par utilisation d'un procédé analytique, le procédé analytique utilisant un jeu de données qui comprend le niveau déterminé de l'au moins un produit d'acide gras oxydé et des données quantitatives provenant d'un ou plusieurs indice(s) clinique(s) comprenant au moins l'un parmi l'âge du sujet, son indice de masse corporelle, et sa concentration d'aspartate transaminase ou d'alanine transaminase.

2. Procédé selon la revendication 1, dans lequel le ou les indice(s) clinique(s) comprend/comprennent au moins deux parmi l'âge du sujet, son indice de masse corporelle, et sa concentration d'aspartate transaminase ou d'alanine transaminase.

3. Procédé selon la revendication 1, dans lequel le jeu de données comprend le niveau déterminé de l'au moins un produit d'acide gras, l'âge du sujet, son indice de masse corporelle, et sa concentration d'aspartate transaminase ou d'alanine transaminase.

4. Procédé selon la revendication 1, dans lequel le procédé analytique comprend l'utilisation d'un modèle d'analyse discriminante linéaire, un algorithme de classification par machines à vecteur de support, un modèle d'élimination de caractéristique récursive, une analyse de prédiction de modèle de micromatrice, un modèle de régression logistique, un algorithme CART, un algorithme FlexTree, un algorithme des forêts aléatoires, un algorithme MART, ou des algorithmes d'apprentissage machine.

5. Procédé selon la revendication 1, dans lequel ledit procédé comprend l'utilisation d'un modèle d'analyse discriminante linéaire ou un modèle de régression logistique, et ledit modèle comprend des termes pour le jeu de données choisis de façon à offrir une évaluation métrique de qualité supérieure à environ 0,8 pour une gravité ou un risque accru d'hépatite graisseuse non alcoolique.

6. Procédé selon la revendication 1, comprenant en outre l'obtention d'une pluralité de scores de risque pour une pluralité d'échantillons obtenus à partir du sujet à une pluralité de temps différents.

7. Procédé selon la revendication 1, dans lequel la signification statistique (valeur p) du niveau d'au moins un produit d'acide gras oxydé chez un sujet ayant une hépatite graisseuse non alcoolique, en comparaison avec un niveau chez un sujet normal, est inférieure à 0,2.

8. Procédé selon la revendication 7, dans lequel la valeur p est inférieure à environ 0,05.

9. Procédé selon la revendication 1, dans lequel l'au moins un produit d'acide gras oxydé est choisi dans l'ensemble constitué par 12-HETE, 15-HETE, 11-HETE, 8-HETE, 9-HETE, 5-HETE, 13-HODE, 9-HODE, 9-oxoODE, 13-oxoODE, et les F2-isoprostanes.

10. Procédé selon la revendication 1, dans lequel l'au moins un produit d'acide gras oxydé est choisi dans l'ensemble constitué par 13-HODE, 9-HODE, 9-oxoODE et 13-oxoODE.

11. Procédé selon la revendication 1, comprenant en outre la comparaison du rapport de l'au moins un produit d'acide gras oxydé sur au moins une molécule de précurseur de produit d'acide gras oxydé.

12. Procédé selon la revendication 11, dans lequel au moins une molécule de précurseur de produit d'acide gras oxydé comprend de l'acide arachidonique ou de l'acide linoléique.

13. Procédé selon la revendication 1, dans lequel le contrôle comprend le score de risque d'un sujet ou tissu normal ou en bonne santé.

14. Procédé selon la revendication 1, dans lequel le procédé analytique pour déterminer le score de risque comprend l'algorithme : score de risque = [-10,051 + 0,0463*âge (ans) + 0,147*IMC (kg/m²) + 0,0293*(AST ou ALT) (UI/l) + 2,658*(rapport du produit d'acide gras oxydé sur le précurseur d'acide gras oxydé (mmol/mol)] * 10.

15. Procédé selon la revendication 1, dans lequel le procédé analytique pour déterminer le score de risque comprend l'algorithme : score de risque = [-10,051 + 0,0463*âge (ans) + 0,147*IMC (kg/m²) + 0,0293*AST (UI/l) + 2,658*rapport HODE-13:LA (mmol/mol)] * 10.

16. Procédé selon la revendication 15, dans lequel un score de risque de 2,2 indique que le sujet présente une NASH ou un risque sensiblement accru de NASH.
